**Europäisches Patentamt**

**European Patent Office**

**Office européen des brevets**

(11) Veröffentlichungsnummer: **0 400 553**
**A1**

## (12) EUROPÄISCHE PATENTANMELDUNG

(21) Anmeldenummer: **90110115.4**

(22) Anmeldetag: **28.05.90**

(51) Int. Cl.5: **C12N 15/82, A01H 5/00, C12N 5/10**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4323.

(30) Priorität: **29.05.89 CH 2000/89**

(43) Veröffentlichungstag der Anmeldung:
**05.12.90 Patentblatt 90/49**

(84) Benannte Vertragsstaaten:
**AT BE CH DE DK ES FR GB GR IT LI LU NL SE**

(71) Anmelder: **SCHWEIZERISCHE EIDGENOSSENSCHAFT EIDGENÖSSISCHE TECHNISCHE HOCHSCHULE (ETH)**
**ETH-Zentrum Rämistrasse 101**
**CH-8092 Zürich(CH)**

(72) Erfinder: **Neuhaus, Gunther, Dr.**
**Birkenstrasse 3**
**CH-8708 Männedorf(CH)**
Erfinder: **Potrykus, Ingo, Prof. Dr.**
**Im Stigler 54**
**CH-4312 Magden(CH)**

(74) Vertreter: **Zumstein, Fritz, Dr. et al**
**Dr. F. Zumstein Dipl.-Ing. F. Klingseisen**
**Bräuhausstrasse 4**
**D-8000 München 2(DE)**

(54) **Verfahren zur Herstellung transgener Pflanzen.**

(57) Gegenstand der vorliegenden Erfindung ist ein neuartiges, reproduzierbares Verfahren zur Einschleusung genetischen Materials in die Zellen der Apikal- und Axialmeristeme von Pflanzen unter Verwendung der Mikroinjektionstechnologie.

Weiterhin betrifft die vorliegende Erfindung die Verwendung des erfindungsgemässen Verfahrens zur Herstellung transgener Pflanzen sowie die mit Hilfe dieses Verfahrens erhältlichen transgenen Pflanzen selbst und deren Nachkommen.

EP 0 400 553 A1

## Verfahren zur Herstellung transgener Pflanzen

Gegenstand der vorliegenden Erfindung ist ein neuartiges, reproduzierbares Verfahren zur Herstellung transgener Pflanzen, das auf der Einschleusung genetischen Materials in die Zellen der Apikal- und Axialmeristeme (Adventivmeristeme) von Pflanzen unter Verwendung der Mikroinjektionstechnologie beruht und welches sich durch seine universelle Anwendbarkeit auszeichnet.

Weiterhin betrifft die vorliegende Erfindung die Verwendung des erfindungsgemässen Verfahrens zur Herstellung transgener Pflanzen sowie die mit Hilfe dieses Verfahrens erhältlichen transgenen Pflanzen selbst und deren Nachkommen.

Für die genetische Manipulation des pflanzlichen Erbgutes mit Hilfe der rekombinanten DNA Technologie stehen mittlerweile eine Vielzahl von Verfahren und Techniken zur Verfügung, die in vielen Laboratorien bereits routinemässig angewendet werden.

Zu den am besten untersuchten und am häufigsten verwendeten Verfahren zählt zweifellos das Agrobacterium-Transformationssystem. Agrobacterium-Zellen besitzen auf ihrem Ti-Plasmid ein grosses DNA-Fragment, die sogenannte T-DNA-Region, das bei der natürlichen Transformation pflanzlicher Zellen in das Pflanzengenom integriert wird.

Dieses natürliche Gen-Transfer-System kann nach Ausführung verschiedener Modifikationen als Gen-Vektor-System für die gezielte Transformation von Pflanzen verwendet werden [Chilton, MD, 1983]. Das Agrobacterium-Transformationssystem hat aber den entscheidenden Nachteil, dass der Wirtsbereich der Agrobakterien auf bestimmte dikotyle Pflanzen sowie auf einige wenige Vertreter der Monokotyledonen (Hemalsteens et al, 1984; Hookas-Van-Slogteren et al., 1984), die aber aus agrarökonomischer Sicht unbedeutend sind, beschränkt ist. Das bedeutet, dass die wichtigsten Kulturpflanzen für einen effektiven Gentransfer nicht zugänglich sind.

Darüberhinaus handelt es sich bei den verwendeten Agrobakterien um Pathogene, die bei ihren Wirtspflanzen charakteristische Krankheitssymptome in Form von krebsartigen Gewebewucherungen auslösen und daher nur unter Wahrung strenger Sicherheitsauflagen im Labor gehandhabt werden dürfen.

Alternative Transformationssysteme, die entwickelt wurden, um diese Nachteile des Agrobacterium Transformationssystems auszugleichen und die auf eine Einschleusung exogener DNA in pflanzliche Protoplasten abzielen, wie der direkte Gentransfer Vektor-freier DNA in Protoplasten (Paszkowski et al., 1984, Potrykus et al., 1986) sowie die Mikroinjektion Vektor-freier DNA in Protoplasten (Spangenberg et al, 1986; Steinbiss and Stabel, 1983; Morikawa and Yamada, 1985) oder Zellen (Nomura and Komamine, 1986), müssen insofern als problematisch angesehen werden, als die Regeneration ganzer Pflanzen aus pflanzlichen Protoplasten einer Vielzahl von Pflanzenspezies, insbesondere aus der Gruppe der Gramineen, zur Zeit noch zahlreiche Probleme mit sich bringt.

Untersuchungen von Yamada et al., 1986, und Toriyma et al., 1986, u.a. zur Regeneration von Reis-Protoplasten sowie von Rhodes et al, 1988 (Mais) und Harris et al, 1988 (Weizen) haben hier erste Fortschritte erkennen lassen.

Ein weiterer Nachteil dieser alternativen Transformations-Systeme betrifft die nach wie vor relativ geringen Transformationsraten, die zur Zeit etwa bei Werten zwischen 1 % und 5 % liegen. Diese niedrigen Transformationsraten machen es weiterhin notwendig, die einzuschleusende DNA mit Markern (z.B. Antibiotika-Resistenzgene) zu versehen, die eine rasche Selektionierung der Transformanten aus der grossen Zahl nichttransformierter Zellen ermöglichen.

Dies bedeutet aber, dass zur Zeit kein befriedigendes Transformations-Verfahren zur Verfügung steht, das eine auch unter kommerziellen Gesichtspunkten effiziente und kostengünstige Produktion transgener Pflanzen mit neuen und nützlichen Eigenschaften erlaubt, insbesondere was die Pflanzen aus der Gruppe der Monocotyledoneae angeht.

Es muss daher als eine vordringliche Aufgabe angesehen werden, Verfahren zu entwickeln, die eine schnelle, effiziente und reproduzierbare Transformation aller Pflanzen, unabhängig von ihrer taxonomischen Stellung und den damit verbundenen Besonderheiten, erlauben und somit eine auch unter kommerziellen Gesichtspunkten effektive und wirtschaftliche Produktion transgener Pflanzen gewährleisten.

In besonderem Masse trifft dies auf Pflanzen aus der Gruppe der Monocotyledoneae zu, insbesondere auf solche aus der Familie Gramineae, in der die aus agrarökonomischer Sicht bedeutendsten Kulturpflanzen wie Weizen, Gerste, Roggen, Hafer, Mais, Reis, Hirse u.a.m. enthalten sind und die daher von ganz besonderem wirtschaftlichem Interesse sind, insbesondere da für die Herstellung transgener monokotyler Pflanzen bisher noch keine befriedigenden Verfahren zur Verfügung stehen.

Erste Ansätze in dieser Richtung liefern verschiedene, erst in jüngster Zeit entwickelte Transformations-Verfahren. Dabei handelt es sich zum einen um die Injektion exogener DNA in die jungen Blütenstände von Roggenpflanzen (de la Pena et al., 1987), zum anderen um eine Agrobacterium-vermittelte Virusinfektion von Mais-Pflanzen mit "Maize Streak Virus" (Grimsley et al., 1987). Doch auch diese neu entwickelten Verfahren sind mit Nachteilen verbunden; so hat sich beispielsweise das zuerst genannte Verfahren als nicht reproduzierbar erwiesen.

Neuhaus et al, 1987 beschreiben ein in vitro Verfahren zur Transformation von Rapspflanzen durch Mikroinjektion von DNA-Lösungen in Embryoide, die in Mikrokultur (in vitro) gehalten und nach erfolgter Transformation zu ganzen Pflanzen regeneriert werden.

Die vorliegende Erfindung kann als eine Weiterentwicklung des bei Neuhaus et al, 1987 beschriebenen Mikroinjektionsverfahrens angesehen werden. Der Haupt-Vorteil des erfindungsgemässen Verfahrens gegenüber diesem bei Neuhaus et al beschriebenen Verfahren liegt in der Verwendung ganzer Pflanzen als Ausgangsmaterial für die Mikroinjektion DNA-haltiger Lösungen. "DNA haltige Lösung" ist hier und im folgenden nicht im chemischen Sinne als echte Lösung zu interpretieren, sondern als eine, die DNA enthaltende Flüssigkeit, die sowohl eine echte Lösung, als auch eine Suspension, Emulsion, etc. sein kann. Durch die Verwendung ganzer Pflanzen kann auf die zum Teil zeit- und damit kostenaufwendige Präparation und Kultivierung geeigneter Embryonal stadien verzichtet werden. Das Arbeiten mit ganzen Pflanzen macht darüberhinaus den zur Zeit noch teilweise komplexen und mit diversen Problemen verbundenen Regenerierungsschritt -abhängig vom verwendeten Pflanzenmaterial- überflüssig.

Im Rahmen der vorliegenden Erfindung ist es jetzt überraschenderweise gelungen, ein in hohem Masse effizientes und in seiner Einfachheit und allgemeinen Anwendbarkeit kaum mehr zu übertreffendes Transformationssystem für Pflanzen, unabhängig von deren taxonomischer Zugehörigkeit und den damit verbundenen Besonderheiten, zu entwickeln, das frei ist von den zuvor genannten Beschränkungen der bisher bekannten Verfahren und das sich somit für eine routinemässige Anwendung in hervorragender Weise geeignet ist.

Im einzelnen handelt es sich dabei um ein Verfahren zur Einschleusung von genetischem Material in Pflanzen, das sich dadurch kennzeichnet, dass man eine DNA-haltige Lösung in die Zellen der zuvor freigelegten Apikal- und/oder Axialmeristeme von Keimpflanzen mikroinjiziert und die mikroinjizierten Keimpflanzen zu ganzen, vollständigen und vorzugsweise fertilen Pflanzen heranzieht. Das Freilegen der Meristeme, das z.B. durch Entfernen oder Auseinanderziehen der Keimblätter erfolgen kann, beeinträchtigt die weitere Entwicklung der Pflanze nicht, sodass man diese nach erfolgter Mikroinjektion wie normale Sämlinge weiterkultivieren kann.

Besonders bevorzugt im Rahmen der vorliegenden Erfindung ist die Verwendung von sog. "Vektorfreier" DNA, d,h. von DNA die keine Sequenzen aufweist, die bekanntermassen mit der Einschleusung oder der Integration von Fremd-DNA in Pflanzen in funktionellem Zusammenhang stehen, wie z.B. die entsprechenden Abschnitte aus den Ti- oder Ri-Plasmiden der Agrobakterien.

Da es sich bei den mit Hilfe dieses Verfahrens erhältlichen primären Transformanten um Chimären handelt, die nur in einem Teil ihrer Zellen das mikroinjizierte genetische Material enthalten, wird für die Herstellung homogen transformierter Pflanzen auf die asexuelle oder sexuelle Nachkommenschaft besagter primärer Transformanten zurückgegriffen.

Besonders bevorzugt im Rahmen der vorliegenden Erfindung ist daher ein Verfahren zur Einschleusung von genetischem Material in Pflanzen, das sich dadurch kennzeichnet, dass man eine DNA-haltige Lösung in die Zellen der zuvor freigelegten Apikal- und/oder Axialmeristeme von Keimpflanzen mikroinjiziert und ausgehend von besagten mikroinjizierten Keimpflanzen oder von Teilen davon ganze, insbesondere aber ganze fertile Pflanzen heranzieht und nach Zwischenschaltung eines oder mehrerer sexueller oder asexueller Vermehrungsschritte die so erhältliche homogen transformierte asexuelle oder sexuelle Nachkommenschaft besagter heterogen transformierter Ausgangspflanzen mit Hilfe bekannter Verfahren selektioniert.

Alternativ dazu können insbesondere bei Pflanzen, die einer Regeneration ausgehend von isolierten Meristemen zugänglich sind, die mikroinjizierten Apikal- und/oder Axialmeristeme entweder unmittelbar nach der erfolgten Mikroinjektion oder aber zu einem beliebigen späteren Zeitpunkt mit oder ohne Selektionsdruck isoliert und zu morphogenenen Kulturen herangezogen werden, aus denen dann wiederum mit Hilfe bekannter Verfahren ganze Pflanzen regeneriert werden können.

Ein weiterer Gegenstand der vorliegenden Erfindung betrifft die Herstellung transgener ganzer Pflanzen, die unter Verwendung des erfindungsgemässen Verfahrens mit einem rekombinanten DNA Molekül transformiert werden, welches in der transformierten Pflanze zu neuen und wünschenswerten Eigenschaften führt sowie die auf diese Weise erzeugten transgenen Pflanzen selbst und deren Nachkommen, sowie Mutanten und Varianten besagter transgener Pflanzen.

Die vorliegende Erfindung betrifft weiterhin Pflanzen, welche aus transformierten Zellen oder sonstigen Pflanzenteilen, die besagtes rekombinantes DNA Molekül enthalten, regeneriert werden sowie deren Samen, darüberhinaus die Nachkommen von Pflanzen, die aus besagtem transgenen Pflanzenmaterial regeneriert worden sind sowie Mutanten und Varianten davon.

Unter den Nachkommen transgener Pflanzen sind im Rahmen dieser Erfindung sowohl auf sexuellem als auch auf asexuellem Wege erzeugte Abkömmlinge der transformierten Mutterpflanze zu verstehen sowie die mit Hilfe von Zellkultur- und/oder Gewebekultur techniken hergestellten Abkömmlinge der mikroinjizierten und isolierten Apikal- und/oder Axialmeristeme.

Ein weiterer Gegenstand der vorliegenden Erfindung betrifft die Verwendung des erfindungsgemässen Transformations-Verfahrens zur schnellen und reproduzierbaren Herstellung transgener Pflanzen mit neuen und wünschenswerten Eigenschaften.

In der folgendenn Beschreibung werden eine Reihe von Ausdrücken verwendet, die in der rekombinanten DNA Technologie, sowie in der Pflanzengenetik gebräuchlich sind. Um ein klares und einheitliches Verständnis der Beschreibung und der Ansprüche sowie des Umfangs, der den besagten Ausdrücken zukommen soll, zu gewährleisten, werden die folgenden Definitionen aufgestellt.

Apikalmeristem: Bildungs- und Teilungszone von Zellen in der Spitzenregion der Sprossachse.

Axial ( = Adventis)meristem: Meristeme, die nicht in Verbindung mit einem Apikalmeristem stehen, sondern aus Geweben der Sprossachse neu gebildet werden [z.B. Blattachselknospen (-meristeme)].

Chimäre: Zelle, Gruppe von Zellen, Gewebe oder Pflanze, zusammengesetzt aus Zellen mit unterschiedlicher genetischer Information ("heterogen transformierte Pflanzen").

Pflanzliches Material: In Kultur oder als solches lebensfähige pflanzliche Teile wie Protoplasten, Zellen, Kallus, Gewebe, Embryonen, Pflanzenorgane, Knospen, Samen, u.a. sowie ganze Pflanzen.

Pflanzenzelle: Strukturelle und physiologische Einheit der Pflanze, bestehend aus einem Protoplasten und einer Zellwand.

Protoplast: Aus Pflanzenzellen oder -geweben isolierte zellwandlose "nackte" Pflanzenzelle mit der Potenz zu einem Zellklon oder einer ganzen Pflanze zu regenerieren.

Zellklon: Aus einer Zelle durch fortlaufende Mitosen entstandene Population von genetisch gleichen Zellen.

Proembryo: Vorläuferstadium des Embryos vor der polaren Differenzierung in die oben angegebenen, für den Embryo charakteristischen Strukturen.

Pflanzengewebe: Gruppe von Pflanzenzellen, die in Form einer strukturellen und funktionellen Einheit organisiert sind.

Pflanzenorgan: Strukturelle und funktionelle Einheit aus mehreren Geweben, wie beispielsweise Wurzel, Stamm, Blatt oder Embryo.

Heterologe(s) Gen(e) oder DNA: Eine DNA Sequenz, die ein spezifisches Produkt oder Produkte kodiert oder eine biologische Funktion erfüllt und die von einer anderen Spezies stammt als derjenigen, in welche das besagte Gen eingeschleust wird; besagte DNA Sequenz wird auch als Fremdgen oder Fremd-DNA bezeichnet.

Homologe(s) Gen(e) oder DNA: Eine DNA Sequenz, die ein spezifisches Produkt oder Produkte kodiert oder eine biologische Funktion erfüllt und die aus der gleichen Spezies stammt, in welche das besagte Gen eingeschleust wird.

Synthetische(s) Gen(e) oder DNA: Eine DNA-Sequenz, die ein spezifisches Produkt oder Produkte kodiert oder eine biologische Funktion erfüllt und die auf synthetischem Wege hergestellt sind.

Vegetationskegel: Zone in der Spitze einer Sprossachse, in der zentral das Bildungsgewebe liegt.

Pflanzen-Promotor: Eine Kontrollsequenz der DNA Expression, die die Transkription jeder beliebigen homologen oder heterologen DNA Gensequenz in einer Pflanze gewährleistet, sofern besagte Gensequenz in operabler Weise mit einem solchen Promotor verknüpft ist.

Terminations-Sequenz: DNA-Sequenz am Ende einer Transkriptions-Einheit, die das Ende des Transkriptionsvorganges signalisiert.

Ueberproduzierender Pflanzen-Promotor (OPP): Pflanzen-Promotor, der in der Lage ist, in einer transgenen Pflanzenzelle die Expression einer jeden in operabler Weise verknüpften funktionalen Gensequenz(en) in einem Ausmass (gemessen in Form der RNA oder der Polypeptidmenge) zu bewirken, das deutlich höher liegt, als dies natürlicherweise in Wirtszellen, die nicht mit besagtem OPP transformiert sind, beobachtet wird.

$3', 5'$ nicht-translatierte Region: Stromabwärts/stromaufwärts der kodierenden Region gelegene DNA-Abschnitte, die zwar in mRNA transkribiert, nicht aber in ein Polypeptid übersetzt werden. Diese Region enthält regulatorische Sequenzen, wie z.B. die Ribosomenbindungsstelle ($5'$) oder das Polyadenylierungssignal ($3'$).

DNA-Expressions-Vektor: Klonierungs-Vehikel, wie z.B. ein Plasmid oder ein Bakteriophage, das alle Signal-Sequenzen enthält, die für die Expression einer inserierten DNA in einer geeigneten Wirtszelle notwendig sind.

DNA-Transfer-Vektor: Transfer-Vehikel, wie z.B. ein Ti-Plasmid oder ein Virus, das die Einschleusung von

4

genetischem Material in eine geeignete Wirtszelle ermöglicht.

Mutanten, Varianten transgener Pflanzen: Spontan oder aber artifiziell, unter Anwendung bekannter Verfahrensmassnahmen, wie z.B. UV-Behandlung, Behandlung mit mutagenen Agentien etc. entstandener Abkömmling einer transgenen Pflanze, der noch die erfindungswesentlichen Merkmale und Eigenschaften der Ausgangspflanze aufweist.

Das erfindungsgemässe Transformations-Verfahren zur direkten Einschleusung genetischen Materials in Pflanzen basiert auf der Mikroinjektion einer DNA-haltigen Lösung in die Zellen der Apikal- und/oder Axialmeristeme von Pflanzen. Besonders bevorzugt ist die Mikroinjektion in die Apikalmeristeme (Vegetationspunkte).

Die Spross- und Wurzelvegetationskegel beherbergen eine Gruppe von Zellen, die als Urmeristemzellen bezeichnet werden und die das Ausgangsmaterial für die Entwicklung des gesamten Pflanzenkörpers darstellen. Diese Urmeristemzellen unterscheiden sich in charakteristischer Weise von den sie umgebenden Zellen. Wie die Embryonalzellen, von denen sie sich unmittelbar ableiten, sind sie verhältnismässig klein und etwa isodiametisch gestaltet. Ihre Zellwände, die meist senkrecht aufeinander stehen sind sehr zart und noch arm an Cellulose. Die Zellen des Urmeristems schliessen dicht aneinander. Der Zellraum ist von dichtem Protoplasma erfüllt, das einen relativ grossen Zellkern beinhaltet. Dieser als Apikalmeristem oder Vegetationspunkt bezeichnete Bereich der Spross- und Wurzelspitzen umfasst nur einige hundert Zellen und ist auf einen Bereich von ca. 100 $\mu$m x 100 $\mu$m konzentriert.

Es wurde nun im Rahmen der vorliegenden Erfindung überraschenderweise gefunden, dass diese im Bereich des Spross- und Wurzelvegetationspunktes konzentrierten Zellen bei Anwendung geeigneter Verfahrensmassnahmen, die weiter unten ausführlich beschrieben sind, einer Mikroinjektion zugänglich sind.

Eine ein- bis mehrmalige Mikroinjektion einer DNA-haltigen Lösung in die Zellen des apikalen Meristems (Vegetationspunkt) von Pflanzen führt zur Bildung transformierter Urmeristemzellen, die sich in der Folge zu transformierten Zellen und Geweben weiterdifferenzieren und somit zur Bildung heterogen transformierter Pflanzen (Chimären) führen. Erfolgt die Mikroinjektion der DNA-haltigen Lösung in einen Bereich innerhalb des Vegetationspunktes, der direkt oder indirekt an der Bildung der Blütenmeristeme beteiligt ist, so kann das eingebaute genetische Material in die Keimbahn der Pflanze gelangen und daher auf die sexuelle Nachkommenschaft der heterogen transformierten Elternpflanzen übertragen werden.

Da die sexuellen Nachkommen aus einer Zelle (der Zygote) entstehen, handelt es sich bei besagten Nachkommen um homogen transformierte Pflanzen, die das über die Mikroinjektion eingeschleuste genetische Material stabil in ihr Genom eingebaut enthalten.

Auf die zuvor beschriebene Weise kann somit die zum Teil unerwünschte Entstehung transgener Chimären vermieden werden.

Bevorzugt im Rahmen dieser Erfindung ist die Mikroinjektion einer DNA-haltigen Lösung in die Vegetationskegel monokotyler Pflanzen, insbesondere aber in die Vegetationskegel von Getreiden und anderen Gräsern, für die bisher kein befriedigendes Transformationssystem zu Verfügung steht.

Im Rahmen der vorliegenden Erfindung ist es jetzt überraschenderweise erstmals gelungen, durch Anwendung spezifischer Verfahrensmassnahmen genetisches Material in vivo gezielt in die Zellen der Apikalmeristeme (Vegetationskegel) von Keimpflanzen zu mikroinjizieren und ausgehend von den so behandelten Pflanzen homogen transformierte Pflanzen heranzuziehen.

Das erfindungsgemässe Verfahren ist nicht auf die Apikalmeristeme beschränkt sondern kann in genau gleicher Weise auch auf die Axialmeristeme der Pflanze angewendet werden.

Die Mikroinjektion der DNA-haltigen Lösung in die Apikal- und/oder Axialmeristeme wird erfindungsgemäss mit Hilfe einer Mikroinjektionsapparatur durchgeführt, die mit manuell oder motorisch steuerbaren Mikromanipulatoren ausgestattet ist.

Der prinzipielle Aufbau sowie die Ausstattung besagter Mikroinjektionseinrichtungen sind in der Literatur vielfach beschrieben. Mikroinjektionsapparaturen werden mittlerweile auch kommerziell als Komplettausrüstung angeboten und gehören bereits zur Standardausrüstung vieler Laboratorien.

Das bevorzugte pflanzliche Ausgansmaterial für die Mikroinjektion sind die Apikal-und/oder Axialmeristeme von Keimlingen. Diese werden nach Durchführung der zuvor beschriebenen Mikroinjektion -abhängig von der verwendeten Pflanzenspezies- entweder direkt in Erde gesetzt und unter Anwendung der üblichen Kultivierungsmassnahmen sehr einfach zu ganzen Pflanzen herangezogen oder aber man isoliert aus den Keimlingen die mikroinjizierten Apikal- und/oder Axialmeristeme oder Teile davon, -sofern Pflanzen betroffen sind, die einer Regeneration aus isolierten Meristemen zugänglich sind-, die dann sehr einfach in vitro zu morphogenenen Kulturen herangezogen und mit Hilfe bekannter Verfahrensmassnahmen zu ganzen Pflanzen regeneriert werden können.

Bei den mit Hilfe des erfindungsgemässen Verfahrens primär erhältlichen Pflanzen handelt es sich um Chimären, d.h. um heterogen transformierte Pflanzen, deren Gewebe nur z.T. das mikroinjizierte genetische Material stabil in ihr Genom eingebaut enthalten. Zur Herstellung homogen transformierter Pflanzen werden daher bevorzugt diejenigen Chimären ausgelesen, die transformierte Keimbahnzellen besitzen und deren sexuelle Nachkommenschaft somit die gewünschte homogene Transformation aufweist. Diese Selektion nach auffälligen Merkmalen kann der Fachmann ohne weiteres vornehmen.

Alternativ dazu können die Chimären in einem in vitro Kultivierungsschritt unter Selektionsdruck kultiviert werden, sofern Pflanzen verwendet werden, die ausgehend von isolierten Apikal- und/oder Axialmeristemen zu ganzen Pflanzen regeneriert werden können. Die freigelegten und mikroinjizierten Apikalmeristeme werden dabei zunächst aus dem Sämling isoliert, in Form von in vitro Kulturen kultiviert und anschliessend zu ganzen Pflanzen regeneriert. Dabei setzt man diese Kulturen vorzugsweise einem gezielten Selektionsdruck aus, sodass nur diejenigen Pflanzen zur Regeneration gelangen, die das injizierte genetische Material exprimieren.

Dieses Alternativverfahren zur Herstellung homogen transformierter Pflanzen, das insbesondere bei den Pflanzen Anwendung finden kann, die sich ausgehend von isolierten Apikal und/oder Axialmeristemen zu ganzen Pflanzen regenerieren lassen, besteht somit in erster Linie in der Identifizierung und Selektionierung transformierter Zellen oder Gewebe aus heterogen transformierten Pflanzen und deren in vitro Regeneration, vorzugsweise in Kultivierungsmedien, die die Bildung adventiver Sprossknospen oder adventiver Embryonen anregt, mit Hilfe bekannter Verfahrensmassnahmen zu ganzen, homogen transformierten Pflanzen.

Verfahren zur in vitro Regeneration transformierten Pflanzenmaterials sind beispielsweise in folgenden Publikationen beschrieben: Paszkowsky and Saul, "Direct Gene Transfer to Plants" in: Methods for Plant Molecular Biology, eds. A & H Weisbach, Academic Press, 1988, pages 447-463; Rogers SG et al, "Gene Transfer in Plants: Production of Transformed Plants Using Ti-Plasmid Vectors", in: Methods for Plant Molecular Biology, eds. A & H Weisbach, Academic Press, 1988, pages 423-436.

Ein wesentlicher Vorteil des erfindungsgemässen Verfahrens ist in dessen universeller Anwendungsbreite zu sehen, die unabhängig ist von der taxonomischen Zugehörigkeit der betreffenden Zielpflanze und den damit verbundenen Besonderheiten und verfahrenstechnischen Schwierigkeiten, wie sie bei Anwendung der bisher zur Verfügung stehenden Transformationsverfahren auftreten, z.B. in Form einer Beschränkung auf bestimmte Dikotyledonen bei Verwendung des Agrobacterium-Transformationssystems oder aber einer bisher noch ungenügenden Regenerierbarkeit monokotyler Pflanzen aus Protoplasten bei Anwendung der direkten Gentransfer-Verfahren.

Diese Universalität des erfindungsgemässen Verfahrens beruht darauf, dass man genetisches Material mit Hilfe einer physikalischen Methode (Mikroinjektion) in eine von jeder Pflanze erhältliche Struktur (Vegetationskegel), unabhängig von deren taxonomischer Zugehörigkeit, überträgt.

Darüberhinaus können durch die direkte Aufzucht von Pflanzen aus Keimlingen die negativen Effekte der 'somaklonalen Variation', die bei anderen Transformations-Verfahren in der Regel auftreten, insbesondere bei solchen, deren Regenerationsschritt über eine Kallusphase verläuft, gänzlich oder aber zumindest teilweise vermieden werden, was für die Belange der Pflanzenzüchtung von ausschlaggebender Bedeutung ist.

Damit ist es im Rahmen der vorliegenden Erfindung jetzt erstmals möglich, unabhängig von den zuvor genannten Beschränkungen, mit hoher Effizienz transgene Pflanzen, insbesondere auch solche aus der Gruppe der Monokotyledoneae, mit neuen und wünschenswerten Eigenschaften zu erzeugen, indem man eine DNA-haltige Lösung in die Zellen der Apikalmeristeme (Vegetationspunkte) und/oder Axialmeristeme von Keimpflanzen mikroinjiziert und die mikroinjizierten Keimlinge oder Teile davon anschliessend unter Anwendung bekannter Kultivierungsmassnahmen zu ganzen, vollständigen Pflanzen heranzieht.

Im einzelnen umfasst das erfindungsgemässe Verfahren die folgenden spezifischen Verfahrensschritte:

a) Keimung von Samen der jeweiligen Zielpflanze und Selektion geeigneter Keimungsstadien;

b) Freilegen des Apikal- und/oder Axialmeristems für die sich anschliessende Mikroinjektion;

c) Mikroinjektion einer DNA-haltigen Lösung enthaltend ein oder mehrere DNA-Fragmente in die Zellen des unter b) freigelegten Meristems;

$d_1$) Anzucht der mikroinjizierten Keimlinge zu vollständigen, heterogen transformierten Pflanzen;

$d_2$) Isolierung und in vitro Regeneration von mikroinjizierten Apikal- und/oder Axialmeristemen ohne Selektionsdruck zu vollständigen, heterogen transformierten Pflanzen;

$d_3$) Isolierung und in vitro Regeneration von mikroinjizierten Apikal- und/oder Axialmeristemen unter Selektionsdruck zu vollständigen, homogen transformierten Pflanzen;

e) Herstellung sexueller oder asexueller Nachkommenschaft der unter Punkt $d_1$) oder $d_2$) erhältlichen heterogen transformierten Pflanzen und Auslese der homogen transformierten Nachkommenschaft.

Für die Bereitstellung geeigneter Keimlinge werden die Samen der jeweiligen Zielpflanzen vorzugsweise unter sterilen Bedingungen gekeimt. Die Keimungsdauer hängt von der jeweiligen Keimungsaktivität des

EP 0 400 553 A1

verwendeten Samenmaterials ab und beträgt in der Regel zwischen 2 und 10 Tagen.

Bevorzugt im Rahmen der vorliegenden Erfindung sind Keimlinge, die sich in einem Entwicklungsstadium befinden, das sich von der beginnenden Kotyledonarentwicklung bis hin zur Sekundärblattentwicklung erstreckt. Besonders bevorzugt sind Keimlinge mit voll entwickelten Kotyledonen.

Im Rahmen der vorliegenden Erfindung ist es vorteilhaft, die Sprossachse des Keimlings kurz zu halten. Dies kann beispielsweise durch geeignete Beleuchtung der keimenden Samen bzw. der jungen Keimlinge erreicht werden. Die Lichtintensität liegt dabei vorteilhafterweise bei ca. 5 bis 145 Einstein$\cdot$s$^{-1}$$\cdot$m$^{-1}$, insbesondere aber bei 45 bis 60 Einstein$\cdot$s$^{-1}$$\cdot$m$^{-1}$.

Wenn die Keimlinge ein bestimmtes, für die Mikroinjektion geeignetes Keimungsstadium erreicht haben, werden zunächst die Vegetationskegel bzw. die Axialmeristeme freigelegt. Beim Sprossvegetationskegel kann dies beispielsweise dadurch erreicht werden, dass man eines oder aber beide Keimblätter entfernt.

Eine weitere Möglichkeit besteht im Auseinanderziehen der Keimblätter bis das apikale Meristem sichtbar ist.

Die Präparation der Vegetationskegel wird vorzugsweise mit Hilfe feiner Skalpelle sowie feiner Nadeln oder fein ausgezogener Pinzetten sowie spezieller Klemmvorrichtungen unter mikroskopischer Kontrolle durchgeführt.

Die Klemmvorrichtung dient insbesondere dazu, die Kotyledonen zu befestigen, wodurch die sich anschliessende Freilegung des Vegetationskegels stark erleichtert wird.

Besonders geeignet für die Verwendung in den erfindungsgemässen Verfahren sind Keimlinge, deren Apikalmeristeme einfach zugänglich sind, wie z.B. die Keimlinge von Sojabohne, Baumwolle, Laubbäumen, Nadelbäumen u.a.

Die auf diese Weise behandelten Keimlinge können anschliessend für die Mikroinjektion verwendet werden.

Die Mikroinjektion der DNA-haltigen Lösung in die Apikal- und/oder Axialmeristeme erfolgt mit Hilfe an sich bekannter Verfahren, die u.a. bei Steinbiss and Stabel, 1983; Neuhaus et al., 1984, 1986, 1987; sowie bei Morikawa and Yamada, 1985 beschrieben sind.

Im einzelnen wird die Mikroinjektion in die einzelnen Zellen des apikalen Meristems unter mikroskopischer Beobachtung sowie unter Verwendung einer spezifischen Mikroinjektionsapparatur durchgeführt, deren Kernstück durch Mikromanipulatoren steuerbare Mikropipetten darstellt.

Der Spitzendurchmesser der Injektionskapillare liegt bei Werten zwischen ≤ 1 $\mu$m und 100 $\mu$m. Ganz besonders geeignet für die Anwendung in dem erfindungsgemässen Verfahren sind Injektionskapillaren mit Spitzendurchmessern zwischen 0,5 $\mu$m und 1 $\mu$m. Man verwendet im Rahmen der vorliegenden Erfindung als Mikroinjektionskapillare vorzugsweise Mikroelektrodenkapillaren mit eingeschmolzenen Glasfilamenten aus Borosilikatglas. Dabei handelt es sich um Kapillaren, deren äusserer Durchmesser zwischen 1,00 mm und 2,00 mm beträgt. Bevorzugt ist ein Durchmesser von 1,2 mm bis 1,5 mm. Selbstverständlich können auch alle anderen, für die Durchführung der Mikroinjektion geeigneten Glasarten oder sonstigen Materialien verwendet werden. Die Mikroinjektionspipette ist in der Regel über eine Druckregulationseinheit, den sog. Mikroinjektor, mit einer Gasquelle, insbesondere einer Stickstoffquelle oder einer Druckluftquelle verbunden, welche einen sehr fein dosierten Uebertritt der Injektionslösung aus der Mikropipette in das pflanzliche Gewebe gewährleistet.

Nach dem Eindringen der Mikropipettenspitze in die Zellen des Apikal- und/oder Axialmeristems erfolgt die Freisetzung der DNA-haltigen Injektionslösung.

Das Injektionsvolumen schwankt in Abhängigkeit von der Zellgrösse zwischen 1 pl und 100 pl pro injizierter Zelle.

Bevorzugt im Rahmen dieser Erfindung ist ein Injektionsvolumen von 10 pl bis 40 pl pro injizierter Zelle.

Um eine möglichst gleichmässige Transformation aller Zellen zu gewährleisten, die am Aufbau der zu injizierenden multizellulären Struktur beteiligt sind, werden diese in Abhängigkeit von der vorhandenen Zellzahl zwischen 1 bis 1000 mal, vorzugsweise aber zwischen 4 bis 60 mal injiziert.

Dies kann beispielsweise dadurch erreicht werden, dass man die zu injizierenden Keimlinge zwischen den verschiedenen Injektionsvorgängen unter der Mikroinjektionseinrichtung dreht, sodass eine möglichst grosse Anzahl von verschiedenen Zellen getroffen wird.

Als DNA-Lösungen, die für eine Mikroinjektion in pflanzliche Zellen geeignet sind, kommen beispielsweise Pufferlösungen in Frage, welche die transformierende DNA in einer geeigneten Konzentration enthalten.

Die im Rahmen dieser Erfindung bevorzugte DNA-Konzentration liegt bei 0,01 $\mu$g/$\mu$l bis 10 $\mu$g/$\mu$l, insbesondere aber bei 0,1 $\mu$g/$\mu$l bis 5 $\mu$g/$\mu$l. Ganz besonders bevorzugt ist eine DNA-Konzentration von 0,5 $\mu$g/$\mu$l bis 1,5 $\mu$g/$\mu$l

Der pH der Pufferlösung kann zwischen Werten von pH 6 bis pH 8 schwanken; erfindungsgemäss

7

bevorzugt ist ein pH-Bereich zwischen pH 7,5 und pH 7,8.

Für die Integration des Fremdgens in die genomische DNA der Pflanzenzelle ist es vorteilhaft, wenn die transformierende DNA von neutralen DNA-Sequenzen (Träger-DNA) flankiert wird. Die die transformierende DNA flankierenden neutralen DNA-Sequenzen können sowohl synthetischen Ursprungs sein als auch durch Behandlung mit geeigneten Restriktionsenzymen aus natürlich vorkommenden DNA-Sequenzen gewonnen werden. So sind beispielsweise als Träger-DNA natürlich vorkommende Plasmide geeignet, die mit einem selektiven Restriktionsenzym geöffnet worden sind.

Die zur Genübertragung hergestellte DNA-Sequenz kann aber auch eine ringförmige Struktur (Plasmidstruktur) haben. Solche Plasmide bestehen aus einem DNA-Strang, in den die transformierende DNA integriert ist.

Ein Beispiel für ein derartiges Plasmid ist das frei erhältliche Plasmid pUC8 (beschrieben bei Messing J und J Vieira, 1982). Weiter sind als Träger-DNA auch Bruchstücke natürlich vorkommender Plasmide verwendbar. Beispielsweise ist als Träger DNA die Deletionsmutante für Gen VI des Cauliflower-Mosaik-Virus einsetzbar.

Die transformierende DNA kann erfindungsgemäss sowohl in Form offener oder geschlossener Ringe (Plasmid-Struktur) vorliegen oder aber bevorzugterweise eine linearisierte Form aufweisen.

Bevorzugt im Rahmen dieser Erfindung ist die Verwendung eines Gemisches aus linearisierter und überspiralisierter Form.

Die optische Kontrolle des gesamten Mikroinjektionsvorganges erfolgt normalerweise unter mikroskopischer Beobachtung. Bevorzugt sind inverse Mikroskope oder Stereomikroskope mit einer Kombination von Auf- und Durchlicht. Die gewählte Vergrösserung hängt dabei von der Grösse des zu injizierenden Materials ab und schwankt zwischen 10 bis 300 facher Vergrösserung. Als Beleuchtung wird vorzugsweise eine Kaltlichtquelle verwendet um eine Wärmeentwicklung zu vermeiden.

Das erfindungsgemässe Verfahren lässt sich somit durch die folgenden Teilschritte charakterisieren:

a) Fixieren der Keimlinge mit Hilfe einer speziellen Haltevorrichtung

b) Injektion der DNA haltigen Lösung in die Zellen des Apikal- und/oder Axialmeristems mit Hilfe der Mikroinjektionskapillare

c) Drehen der Keimlinge und erneute Injektion.

Die Keimlinge werden nach erfolgter Mikroinjektion entweder direkt in Erde übertragen oder aber alternativ dazu in ein geeignetes Kulturmedium überführt.

Die sich an die Mikroinjektion anschliessende Kultivierung der mikroinjizierten Keimlinge entspricht entweder einer ganz normalen Anzucht von Keimlingen in Erde, wie sie routinemässig auf dem Gebiet der Pflanzenzüchtung durchgeführt wird, oder aber sie wird in spezfischen Kultivierungsmedien durchgeführt, die für die weitere Entwicklung der Keimlinge bis hin zur vollständig ausgebildeten Pflanze geeignet sind.

Besonders bevorzugt im Rahmen dieser Erfindung ist die direkte Uebertragung der mikroinjizierten Keimlinge in Erde.

Die auf diese Weise aus den mikroinjizierten Keimlingen gebildeten Pflänzchen können dann im Gewächshaus in gleicher Weise wie normale Sämlinge weiterbehandelt werden. Es entwickeln sich dann Pflanzen, die nur in einem Teil ihrer Zellen das mikroinjizierte genetische Material enthalten und die üblicherweise als Chimären bezeichnet werden.

Für die Herstellung reinerbiger, homozygoter Transformanten stehen insbesondere zwei alternative Wege zur Verfügung.

Bei Pflanzen die einer Selbstbestäubung zugänglich sind, d.h. die keine Selbstinkompatibilitäten aufweisen, können homozygote Linien durch wiederholte Selbstbestäubung von primären Transformanten (Chimären) und Herstellung von Inzuchtlinien gewonnen werden. Im Unterschied zu homozygoten, diploiden Transformanten, in denen das integrierte Gen auf homologen Chromosomen vorliegt und die aufgrund der dominanten Vererbung dieses Gens den Mendelschen Gesetzen gehorchen, führt eine Selbstung der mikroinjizierten Chimären zu einer heterozygoten Situation in Bezug auf das integrierte Gen (Monohybrid). Erst im Zuge der nachfolgenden weiteren Selbstbestäubungen der Filialgenerationen kommt man schliesslich zu homozygoten Linien in Bezug auf das eingeschleuste Fremdgen.

Die Samen, die man aufgrund dieser Kreuzungen erhält werden jeweils auf geeigneten Selektionsmedien gekeimt, um so die nach den Mendelschen Gesetzen vererbten Merkmale herauszuselektionieren. Diese Inzuchtlinien wiederum können dann zur Entwicklung von Hybriden verwendet werden, sofern diese primären Transformanten transformierte Keimbahnzellen enthalten.

Auch Pflanzen, die einer Selbstbestäubung nicht unmittelbar zugänglich sind, sei es dass die Sexualorgane auf unterschiedlichen Pflanzen vorliegen oder aber die Selbstkreuzung aus irgenwelchen Gründen nicht zur Produktion normaler Samen führt, können zur Herstellung von Inzuchtlinien verwendet werden. In diesem Fall werden Pflanzen, die einen hohen Verwandschaftsgrad aufweisen miteinander gekreuzt, was letztlich ebenfalls zum angestrebten Ziel führen kann, nämlich der Bereitstellung von Inzuchtlinien für die Hybridzüchtung.

Die hier beispielhaft angeführten Verfahren zur Herstellung homozygoter Pflanzen sind dem Fachmann auf dem Gebiet der Pflanzenzüchtung bekannt und z.B. in folgender Publikation im Detail beschrieben:

"Breeding Field Crops, Third Edition, JM Poehlman, AVI Publishing Company, Inc., Westport, Connecticut, 1987"

Bei Pflanzen, die einer Regeneration z.B. über eine sekundäre Embryogenese oder Adventivsprossbildung zugänglich sind, ist es möglich transformierte Einzelzellen oder aber transformierte Gewebe aus den heterogen transformierten Pflanzen zu isolieren und diese in vitro unter Selektionsdruck zu reinen transgenen Pflanzen zu regenerieren.

Im einzelnen kann dabei z.B. so vorgegangen werden, dass im Anschluss an die Mikroinjektion eine in vitro Kultivierung des behandelten Apikal- oder Axialmeristems durchgeführt wird und zwar in Kultivierungsmedien, die für die Regeneration der Zellen oder Gewebe bis hin zur vollständig ausgebildeten Pflanze geeignet sind.

Die Zusammensetzung dieser Medien, die dem Fachmann auf diesem Gebiet bestens bekannt ist, zeichnet sich in erster Linie dadurch aus, dass sie vor allem die für das Wachstum und die Entwicklung der pflanzlichen Zelle essentiellen mineralischen Stoffe bzw. Elemente in ihren Verbindungen (Makroelemente) enthalten, wie z.B. Stickstoff, Phosphor, Schwefel, Kalium, Calcium, Magnesium und Eisen, neben den sogenannten Spurenelementen (Mikroelemente) wie z.B. Natrium, Zink, Kupfer, Mangan, Bor, Vanadium, Selen und Molybdän. Während die Makroelemente in der Regel in Mengen zwischen 10 mg/l und einigen Hundert mg/l vorliegen, beträgt die Konzentration der Mikroelemente im allgemeinen maximal einige mg/l.

Weitere Bestandteile besagter Medien umfassen eine Reihe von essentiellen Vitaminen wie Nikotinsäure, Pyridoxin, Thiamin, Inosit, Biotin, Liponsäure, Riboflavin, Ca-Pantothenat u.a.m., eine geeignete leicht assimilierbare Kohlenstoffquelle, wie beispielsweise Saccharose oder Glucose, sowie verschiedene Wachstumsregulatoren in Form natürlicher oder synthetischer Phytohormone aus der Klasse der Auxine und Cytokinine im Konzentrationsbereich von 0,01 mg/l bis 20 mg/l, vorzugsweise von 0.05 mg/l bis 10 mg/l und ganz besonders bevorzugt von 0.1 mg/l bis 1.2 mg/l.

Dabei ist erfindungsgemäss besonders darauf zu achten, dass sich die Zusammensetzung der Medien sowie die Konzentrationsverhältnisse ihrer einzelnen Komponenten in Abhängigkeit vom Entwicklungsstand der Regeneranten ändern kann. Dies trifft in erster Linie auf die verwendeten Wachstumsregulatoren zu, die in einem ausgewogenen Verhältnis zueinander eingesetzt werden müssen, sodass, falls dies gewünscht wird, ein kontrollierter Ablauf der Regeneration gewährleistet ist.

Eine generelle Faustregel lässt sich in diesem Zusammenhang nicht angeben, da sowohl die individuellen Konzentrationen als auch die Konzentrtionsverhältnisse von Auxin/Cytokinin je nach verwendetem Pflanzenmaterial sehr unterschiedlich sein können. Die Vorgehensweise zur Ermittlung geeigneter Wuchsstoffkonzentrationen sowie geeigneter Konzentrationsverhältnisse der einzelnen Wuchsstoffe untereinander sind dem Fachmann auf diesem Gebiet hinlänglich bekannt.

Bevorzugt im Rahmen dieser Erfindung ist ein Konzentrationsbereich der Phytohormone aus der Klasse der Auxine und der Cytokinine der jeweils zwischen 0,01 mg/l und 20 mg/l, vorzugsweise zwischen 0.05 mg/l bis 10 mg/l und insbesondere zwischen 0,1 mg/l und 1,2 mg/l liegt.

Die Kulturmedien sind darüberhinaus durch Zusatz von Zuckeralkoholen (beispielsweise Mannit), Zucker (beispielsweise Glucose) oder Salzionen osmotisch stabilisiert und sind auf pH-Werte von 4,5 bis 7,5, vorzugsweise von 5,2 bis 6,5, eingestellt.

Neben der Verwendung synthetischer Medien mit einer genau definierten Zusammensetzung aus verschiedenen Einzelkomponenten bekannter Konzentration können auch komplexe Nährmedien verwendet werden, die ganz oder aber zumindest teilweise aus natürlichen Komponenten zusammengesetzt sind. In diesem Zusammenhang sei beispielhaft auf Kartoffelextrakt, Kokosnussmilch sowie auf flüssiges Endosperm verwiesen, das durch Absaugen aus den Samenanlagen von Pflanzen gewonnen werden kann.

Während die ersten Kultivierungsschritte, die sich unmittelbar an die Mikroinjektion anschliessen, vorzugsweise in festen Kulturmedien durchgeführt werden, kann im weiteren Verlauf der Regeneration wahlweise sowohl auf flüssige als auch auf feste Kulturmedien, die durch Zusatz eines der üblicherweise in der mikrobiologischen Technik verwendeten Verfestigungsmittel hergestellt werden können, oder aber auf eine Kombination aus festen und flüssigen Medien zurückgegriffen werden.

Geeignete Verfestigungsmittel umfassen beispielsweise Agar, Agarose, Alginat, Pektinat, Gelrite®, Gelatine u.a. Bevorzugt sind Agar und Agarose, insbesondere Agar und Agarose vom "Low Melting Type" (LMT).

Bei der Verwendung flüssiger Medien kann es gegebenenfalls zweckmässig sein, dem Medium ein osmotisch neutrales Verdickungsmedium zuzusetzen, sodass die Dichte des Mediums zunimmt, ohne dadurch den osmotischen Wert zu erhöhen. Dies führt dazu, dass die Regeneranten auf dem Medium aufschwimmen und somit ein optimaler Gasaustausch mit der umgebenden Atmosphäre gewährleistet ist. Bevorzugt im Rahmen dieser Erfindung sind synthetische Polymere, so z.B. Co-Polymere aus Saccharose und Epichlorhydrin mit einem Molekulargewicht zwischen 70 000 und 400 000, ohne jedoch darauf beschränkt zu sein.

In einer spezifischen Ausführungsform des erfindungsgemässen Verfahrens werden die auf die zuvor beschriebene Weise mikroinjizierten Meristeme unmittelbar nach erfolgter Mikroinjektion freigelegt und vorzugsweise auf handelsübliche Mikrokultivierungsplatten transferiert und zwar in oder auf Medien, die eine für die in vitro Kultivierung dieser meristematischen Gewebe geeignete Zusammensetzung aufweisen.

Die Kultivierung der mikroinjizierten Meristeme wird vorzugsweise auf einem Festmedium durchgeführt, das ein Geliermittel ausgewählt aus der Gruppe bestehend aus Agar, Agarose, Alginat, Pektinat, Gelrite® oder Gelatine als Zusatz enthält.

Die Kultivierungsdichte liegt vorzugsweise zwischen 1 Meristem/µl und 50 Meristemen/µl Kulturmedium, ganz besonders bevorzugt aber zwischen 1 Meristem/µl und 5 Meristemen/µl Kulturmedium. Die gewählte Kultivierungsdichte ist abhängig von der Grösse und dem Entwicklungsstand der verwendeten Meristeme.

Es erweist sich im Rahmen dieser Erfindung als vorteilhaft, wenn die Kultivierung der mikroinjizierten Apikal- und oder Axialmeristeme zumindest zeitweise in einem 2 Kompartimentensystem durchgeführt wird, das als feuchte Kammer fungiert, wobei das äussere Kompartiment beispielsweise von Wasser, von Kulturmedium oder von einer wässrigen Mannit-Lösung gebildet wird, während sich die Meristeme im innern Kompartiment befinden.

Nach einer Kultivierungsdauer von 5 bis 15 Tagen, vorzugsweise aber nach ca. 10 Tagen, werden die sich entwickelnden Apikal- und/oder Axialmeristeme auf ein selektives Kulturmedium übertragen, auf welchem die isolierten Meristeme einem positiven Selektionsdruck ausgesetzt werden, sodass nur diejenigen Zellen überleben und sich weiterentwickeln können, die aufgrund der vorangegangenen Transformation in der Lage sind, diesem Selektionsdruck zu widerstehen. Alle nicht transformierten Zellen sterben dagegen ab oder werden von den transformierten Zellen überwachsen.

Als selektive Medienzusätze können beispielsweise Herbizide, Insektizide, Fungizide sowie andere zelltoxische Verbindungen verwendet werden, wie z.B. verschiedene Antibiotika oder andere Biozide. Daneben können aber auch andere Parameter als selektives Agens wirken, wie z.B. Salzkonzentration, $O_2$-Konzentration, Licht, pH- usw., abhängig vom jeweils verwendeten und in die Pflanze eingeschleusten Selektivmarker.

Wenn die auf die zuvor beschriebene Weise mikrokultivierten Meristeme ein bestimmtes Entwicklungsstadium erreicht haben, in der Regel nach 10 bis 30 Tagen, insbesondere aber nach 10 bis 20 Tagen werden sie vorteilhafterweise auf feste Medien transferiert, die gegebenenfalls von einem Flüssigmedium überlagert sein können. In der Regel handelt es sich dabei um die üblicherweise für die Regeneration von Pflanzen verwendeten Kulturmedien, die aufgrund ihres ausgewogenen Gehaltes an Phytohormonen, insbesondere aus der Klasse der Auxine und Cytokinine, eine vermehrte adventive Sprossregeneration oder aber eine vermehrte adventive Embryogenese hervorrufen. Besonders bevorzugt sind Auxin- und Cytokininkonzentrationen von 0,01 mg/l bis 20 mg/l und ganz besonders bevorzugt von 0.1 mg/l bis 1.0 mg/l, wie sie z.B. in den im folgenden im Detail beschriebenen T3-, T4- und T5-Medien vorliegen.

Nach einer Gesamt-Kultivierungsdauer von ca. 4 bis 8 Wochen, je nach verwendetem Pflanzenmaterial können diese auf Festmedien übertragen werden, die keine oder aber nur eine geringe Hormonkonzentration aufweisen und hier bis zur Ausbildung von kleinen Pflänzchen (Keimlinge) weiterkultiviert werden.

Um eine rasche Regeneration der Pflanzen zu unterstützen, kann es vorteilhaft sein, eine Induktion der Wurzelbildung auszulösen, z.B. durch Uebertragung der Embryonen auf an sich bekannte Wurzel-induzierende Medien [vgl. z.B. Medium T5].

Die auf diese Weise gebildeten transgenen Pflanzen können dann in Erde gepflanzt und in gleicher Weise wie normale Sämlinge im Gewächshaus weiterbehandelt werden.

Das zuvor beschriebene Verfahren zur Herstellung transgener Pflanzen mit Hilfe der Mikroinjektionstechnologie ist mit allen seinen Teilschritten Bestandteil der vorliegenden Erfindung.

Das erfindungsgemässe Verfahren ist universell auf alle Pflanzen anwendbar, die Samen und Keimlinge ausbilden. Es umfasst sowohl die Mikroinjektion von natürlichen DNA-Sequenzen als auch von artifiziell mit Hilfe der rekombinanten DNA-Technologie erzeugten hybriden Genkonstruktionen.

In erster Linie umfasst vom breiten Umfang der vorliegenden Erfindung sind rekombinante DNA-Moleküle, die DNA-Sequenzen enthalten, welche bei den Transformanten zu nützlichen und wünschenswerten Eigenschaften führen.

Dabei handelt es sich vorzugsweise um rekombinante DNA-Moleküle, die eine oder mehrere Gensequenzen beinhalten, die eine nützliche und wünschenswerte Eigenschaft kodieren und die unter der regulatorischen Kontrolle von in Pflanzen aktiven Expressionssignalen stehen, sodass eine Expression besagter Gensequenzen in der transformierten pflanzlichen Zelle gewährleistet ist.

Für die Verwendung in dem erfindungsgemässen Verfahren sind somit in erster Linie alle diejenigen Gene geeignet, die in der pflanzlichen Zelle exprimiert werden und die der Pflanze eine nützliche und/oder gewünschte Eigenschaft verleihen, wie z.B. eine erhöhte Resistenz gegenüber Pathogenen (beispielsweise gegenüber phytophathogenen Pilzen, Bakterien, Viren etc:), eine Resistenz gegenüber Chemikalien [beispielsweise gegenüber Herbiziden (wie z.B. Triazinen, Sulfonylharnstoffen, Imidazolinonen, Triazolpyrimidinen, Bialaphos, Glyphosate, etc.), Insektiziden oder anderen Bioziden]; Resistenz gegenüber nachteiligen (endaphischen oder atmosphärischen) Umwelteinflüssen (beispielsweise gegenüber Hitze, Kälte, Wind, besondere, extreme Bodenbeschaffenheit, Feuchtigkeit, Trockenheit, osmotischer Stress etc.); oder aber zu einer erhöhten oder aber qualitativ besseren Bildung von Reserve- oder Speicherstoffen in Blättern, Samen, Knollen, Wurzel, Stengeln, etc. führen. Zu den wünschenswerten Substanzen, die von transgenen Pflanzen produziert werden können, zählen beispielsweise Proteine, Stärken, Zucker, Aminosäuren, Alkaloide, Riechstoffe, Farben, Fette, etc.

Ebenso können in dem erfindungsgemässen Verfahren Gene eingesetzt werden, die pharmazeutisch akzeptable Wirksubstanzen, wie z.B. Alkaloide, Steroide, Hormone, Immunmodulatoren, und andere physiologisch aktive Substanzen kodieren.

Eine Resistenz gegenüber Cytotoxinen kann beispielsweise durch Übertragung eines Gens erreicht werden, das in der Lage ist, bei der Expression in der pflanzlichen Zelle ein Enzym zur Verfügung zu stellen, welches das Cytotoxin detoxifiziert, wie z.B. die Neomycinphosphotransferase Typ II oder die Aminoglycosidphosphotransferase Typ IV, die zu einer Detoxifikation von Kanamycin, Hygromycin und anderen Aminoglykosidantibiotika beitragen oder eine Glutathion-S-Transferase, Cytochrom P-450 oder andere katabolisch wirksame Enzyme, von denen bekannt ist, dass sie Triazine, Sulfonylharnstoffe oder andere Herbizide detoxifizieren. Eine Resistenz gegenüber Cytotoxinen kann auch durch ein Gen vermittelt werden, das in einer Pflanze eine bestimmte Form eines 'Zielenzyms' (Angriffspunkt der Cytotoxinwirkung) exprimiert, die resistent ist gegen die Aktivität des Cytotoxins, wie z.B. eine Variante der Acetohydroxysäuresynthase, die gegenüber der inhibitorischen Wirkung von Sulfonylharnstoffen, Imidazolinonen oder anderen Herbiziden, die mit diesem spezifischen Stoffwechselschritt interagieren, insensitiv ist; oder eine Variante der EPSP Synthase, die sich gegenüber der Hemmwirkung von Glyphosate als insensitiv erweist. Es kann sich als vorteilhaft erweisen, diese veränderten Zielenzyme in einer Form zu exprimieren, die ihren Transport in das korrekte zelluläre Kompartiment, wie z.B. im obigen Fall in die Chloroplasten, erlaubt.

In bestimmten Fällen kann es von Vorteil sein, die Genprodukte in die Mitochondrien, die Vakuolen, das endoplasmatische Retikulum oder in andere Zellregionen, möglicherweise sogar in die interzellulären Räume (Apoplasten) zu dirigieren.

Eine Resistenz gegenüber bestimmten Pilzklassen kann beispielsweise durch die Einschleusung eines Gens erreicht werden, das Chitinase in den pflanzlichen Geweben exprimiert. Zahlreiche pflanzenpathogene Pilze enthalten Chitin als integralen Bestandteil ihre Hyphen- und Sporenstrukturen, so z.B. die Basidiomyceten (Brand- und Rostpilze), Asomyceten und Fungi Imperfecti (einschliesslich Alternaria und Bipolaris, Exerophilum turcicum, Colletotricum, Gleocercospora und Cercospora). Chitinase ist in der Lage das Mycelwachstum bestimmter Pathogene in vitro zu hemmen. Ein pflanzliches Blatt oder eine Wurzel, die Chitinase konstruktiv oder aber als Antwort auf das Eindringen eines Pathogens exprimiert, sollte somit gegen den Angriff einer grossen Zahl verschiedener Pilze geschützt sein. Je nach Situation kann eine konstitutive Expression vorteilhaft sein im Vergleich zu einer induzierbaren Expression, die bei zahlreichen Pflanzen als normale Reaktion auf einen Pathogenbefall auftritt, da die Chitinase unmittelbar in hoher Konzentration vorliegt, ohne dass eine lag-Phase für die Neusynthese abgewartet werden muss.

Eine Resistenz gegenüber Insekten kann beispielweise durch ein Gen übertragen werden, das ein Polypeptid kodiert, welches toxisch ist für Insekten und/oder deren Larven, wie z.B. das kristalline Protein von Bacillus thuringiensis [Barton et al, 1987; Vaeck et al, 1987]. Eine zweite Klasse von Proteinen, die eine Insektenresistenz vermitteln, sind die Proteaseinhibitoren. Proteaseinhibitoren bilden einen gewöhnlichen Bestandteil von pflanzlichen Speicherstrukturen (Ryan, 1973). Es konnte nachgewiesen werden, dass ein aus Sojabohnen isolierter und gereinigter Bowman-Birk Proteaseinhibitor die Darmprotease von Tenebrio-Larven hemmt (Birk et al, 1963). Das Gen, welches den Trypsininhibitor aus der Kuherbse kodiert, ist bei Hilder et al, (1987) beschrieben.

Ein Gen, das einen Proteaseinhibitor kodiert, kann in einem geeigneten Vektor unter die Kontrolle eines Pflanzenpromotors, insbesondere eines konstitutiven Promotors, wie z.B. den CaMV 35S Promotor [der bei Odell et al, (1985) beschrieben ist], gebracht werden. Das Gen, beispielsweise die kodierende Sequenz des Bowman-Birk Proteaseinhibitors aus der Sojabohne, kann mit Hilfe der bei Hammond et al, (1984) beschriebenen cDNA Klonierungsmethode erhalten werden.

Eine weitere Möglichkeit zur Herstellung eines Proteaseinhibitors besteht in dessen synthetischer Herstellung, sofern dieser weniger als 100 Aminosäuren aufweist, wie z.B. der Trypsininhibitor der Limabohne. Die kodierende Sequenz lässt sich durch Zurückübersetzen der Aminosäuresequenz voraussagen. Zusätzlich werden an beiden Enden Restriktionsschnittstellen eingebaut, die für den jeweils gewünschten Vektor geeignet sind. Das synthetische Gen wird durch Synthese überlappender Oligonukleotidfragmente von 30 bis 60 Basenpaaren hergestellt, indem diese zunächst einer Kinasereaktion unterworfen, dann miteinander verknüpft (Maniatis et al, 1982) und schliesslich in einem passenden Vektor kloniert werden. Mit Hilfe der DNA Sequenzierung kann dann ein Klon, der das Insert in einer korrekten Orientierung aufweist, identifiziert werden. Für die Enschleusung in die Meristeme wird vorzugsweise isolierte Plasmid-DNA verwendet.

Ebenfalls umfasst von der vorliegenden Erfindung sind Gene, die pharmazeutisch aktive Bestandteile kodieren, z.B. Alkaloide. Steroide. Hormone und andere physiologisch aktive Substanzen sowie Flavine, Vitamine und Farbstoffe. Zu den Genen, die im Rahmen dieser Erfindung in Betracht gezogen werden, gehören daher, ohne jedoch darauf beschränkt zu sein, pflanzenspezifische Gene wie z.B. das Zeingen (Wienand et al, 1981), Säuger-spezifische Gene wie das Insulingen, das Somatostatingen, die Interleucingene, das t-PA Gen (Pennica et al, 1983) etc., oder aber Gene mikrobiellen Ursprungs wie das NPT II Gen sowie synthetische Gene wie das Insulingen (Itakura et al, 1975).

Neben natürlicherweise vorkommenden Genen, die eine nützliche und wünschenswerte Eigenschaft kodieren, können im Rahmen dieser Erfindung auch Gene verwendet werden, die zuvor mit Hilfe chemischer oder gentechnologischer Methoden gezielt modifiziert wurden.

Weiterhin sind von dem breiten Konzept der vorliegenden Erfindung auch solche Gene umfasst. die vollständig durch chemische Synthese hergestellt werden.

Weiterhin geeignet für die Verwendung in dem erfindungsgemässen Verfahren sind sonstige nützliche DNA-Sequenzen, insbesondere nicht-kodierende DNA-Sequenzen mit regulatorischen Funktionen. Diese nicht-kodierenden DNA-Sequenzen sind beispielsweise in der Lage die Transkription einer assoziierten DNA-Sequenz in pflanzlichen Geweben zu regulieren.

In diesem Zusammenhang ist z.B. der Promotor des hps70 Gens zu nennen, der durch einen Hitzeschock induziert werden kann (Spena et al, 1985); die 5'-flankierende Sequenz der nukleär kodierten Sequenz der kleinen Untereinheit des Ribulose-1,5-biphosphatcarboxylase (RUBISCO) Gens [Morelli et al, 1985] oder des Chlorophyll a b Bindungsproteins, die durch Licht induzierbar sind; oder die 5'-flankierende Region des Alkoholdehydrogenase (adh1-s)Gens aus Mais, die unter anaeroben Bedingungen die Transkription eines assoziierten Reportergens [z.B. Chloramphenicolacetyltransferase-Gen (CAT)] auslöst (Howard et al, 1977).

Eine weitere Gruppe regulierbarer DNA-Sequenzen betrifft chemisch regulierbare Sequenzen, die z.B. in den PR-("pathogenesis related proteine")Proteingenen des Tabaks vorliegen und mit Hilfe chemischer Regulatoren induzierbar sind.

Die zuvor beispielhaft genannten regulierbaren DNA-Sequenzen können sowohl natürlichen als auch synthetischen Ursprungs sein oder aber aus einem Gemisch von natürlichen und synthetischen DNA-Sequenzen bestehen.

Als Gene oder DNA-Sequenzen, die im Rahmen der vorliegenden Erfindung Verwendung finden können, kommen somit sowohl homologe als auch heterologe Gen(e) oder DNA sowie synthetische Gen(e) oder DNA gemäss der im Rahmen der vorliegenden Erfindung gemachten Definition in Betracht.

Die DNA-Sequenz kann ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und oder synthetischer DNA.

In diesem Fall kann die cDNA aus demselben Gen stammen wie die genomische DNA oder aber, sowohl die cDNA als auch die genomische DNA können aus verschiedenen Genen stammen. In jedem Fall können aber sowohl die genomische DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die verschiedenen Stämmen oder Varietäten derselben Art oder verschiedenen Spezies derselben Gattung angehören, oder aber von Organismen, die mehr als einer Gattung derselben oder einer anderen taxonomischen Einheit (Reich) angehören, abstammen.

Um die Expression besagter Strukturgene in der pflanzlichen Zelle zu gewährleisten, ist es vorteilhaft, wenn die kodierenden Gensequenzen zunächst in operabler Weise mit in pflanzlichen Zellen funktionsfähigen Expressions-Sequenzen verknüpft werden.

Die hybriden Genkonstruktionen im Rahmen der vorliegenden Erfindung enthalten somit neben dem (den) Strukturgen(en) Expressions-Signale, die sowohl Promotor- und Terminator-Sequenzen als auch weitere regulatorische Sequenzen der 3' und 5' nicht-translatierten Regionen miteinschliessen. Jeder Promotor und jeder Terminator, der in der Lage ist, eine Induktion der Expression einer kodierenden DNA-Sequenz (Strukturgen) zu bewirken, kann als Bestandteil der hybriden Gensequenz verwendet werden. Besonders geeignet sind Expressionssignale, die aus Genen von Pflanzen oder Pflanzenviren stammen. Beispiele geeigneter Promotoren und Terminatoren sind z.B. solche der Nopalin-Synthase-Gene (nos), der Octopin-Synthase-Gene (ocs) sowie der Cauliflower Mosaik Virus Gene (CaMV) oder aber homologe DNA-Sequenzen, die noch die charakteristischen Eigenschaften der genannten Expressionssignale aufweisen.

Bevorzugt im Rahmen dieser Erfindung sind die 35S und 19S Expressions-Signale des CaMV Genoms oder deren Homologe, die mit Hilfe molekularbiologischer Methoden, wie sie z.B. bei Maniatis et al., 1982 beschrieben sind, aus besagtem Genom isoliert und mit der kodierenden DNA-Sequenz verknüpft werden können.

Unter Homologen der 35S und 19S Expressionssignale sind im Rahmen dieser Erfindung Sequenzen zu verstehen, die trotz geringer Sequenzunterschiede den Ausgangssequenzen im wesentlichen homolog sind und die nach wie vor die gleiche Funktion wie diese erfüllen.

Als Ausgangsmaterial für die 35S-Transkriptionskontroll-Sequenzen kann erfindungsgemäss z.B. das Scal-Fragment des CaMV Stammes "S", das die Nukleotide 6808-7632 der Genkarte umfasst (Frank G et al., 1980), verwendet werden.

Die 19S Promotor- und 5' nicht-translatierte Region befindet sich auf einem Genomfragment zwischen der PstI Stelle (Position 5386) und der HindIII-Stelle (Position 5850) der CaMV Genkarte (Hohn et al., 1982). Die entsprechende Terminator- und 3' nichttranslatierte Region liegt auf einem EcoRV'BgIII-Fragment zwischen Position 7342 und 7643 des CaMV Genoms.

Weiterhin bevorzugt im Rahmen dieser Erfindung sind die Expressionssignale des CaMV Stammes CM 1841, dessen komplette Nukleotidsequenz bei Gardner RC et al, 1981 beschrieben ist.

Ein weiterer wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz, welche ein gewünschtes Genprodukt kodiert, verknüpft ist, in der Lage sein, die Expression besagter Gensequenz zu vermitteln.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bisphosphat-Carboxylase aus Sojabohnen [Berry-Lowe et al., J. Molecular and App. Gen., 1:483-498 (1982)] sowie den Promotor des Chlorophyll-a/b-Bindungsproteins ein. Diese beiden Promotoren sind dafür bekannt, dass sie in eukaryontischen Pflanzenzellen durch Licht induziert werden [siehe z.B. Genetic Engineering of Plants, an Agricultural Perspective, A. Cashmore, Plenum, New York 1983) Seite 29-38; Coruzzi G. et al., The Journal of Biological Chemistry, 258: 1399 (1983) und Dunsmuir, P. et al., Journal of Molecular and Applied Genetics, 2: 285 (1983)].

Die verschiedenen DNA-Sequenzabschnitte können mit Hilfe an sich bekannter Methoden miteinander zu einer vollständigen kodierenden DNA-Sequenz verknüpft werden. Geeignete Methoden schliessen beispielsweise die in vivo Rekombination von DNA-Sequenzen, die homologe Abschnitte aufweisen sowie die in vitro Verknüpfung von Restriktionsfragmenten mit ein.

Als Klonierungsvektoren verwendet man im allgemeinen Plasmid- oder Virus-(Bakteriophage)-Vektoren mit Replikations- und Kontrollsequenzen, die von Spezies stammen, die mit der Wirtszelle kompatibel sind.

Der Klonierungsvektor trägt in der Regel einen Replikationsursprung, ausserdem spezifische Gene, die zu phaenotypischen Selektionsmerkmalen in der transformierten Wirtszelle führen, insbesondere zu Resistenzen gegenüber Antibiotika oder gegenüber bestimmten Herbiziden. Die transformierten Vektoren können anhand dieser phaenotypischen Marker nach einer Transformation in einer Wirtszelle selektiert werden.

Selektierbare phaenotypische Marker, die im Rahmen dieser Erfindung Anwendung finden können, umfassen beispielsweise, ohne das dies eine Limitierung des Erfindungsgegenstandes darstellt, Resistenzen gegen Ampicillin, Tetracyclin, Hygromycin, Kanamycin, Metotrexat, G418 und Neomycin.

Als Wirtszellen kommen im Rahmen dieser Erfindung Prokaryonten in Frage, einschliesslich bakterieller Wirte, wie z.B. A.tumefaciens, E.coli, S.typhimurium und Serratia marcescens, sowie Cyanobakterien. Ferner können auch eukaryontische Wirte wie Hefen, mycelbildende Pilze und Pflanzenzellen im Rahmen dieser Erfindung verwendet werden.

Das Einspleissen der erfindungsgemässen hybriden Genkonstruktion in einen geeigneten Klonierungsvektor erfolgt mit Hilfe von Standardmethoden wie sie z.B. bei Maniatis et al., 1982 beschrieben sind.

Dabei wird in der Regel der Vektor und die einzuspleissende DNA Sequenz zunächst mit geeigneten Restriktions-Enzymen geschnitten. Geeignete Restriktionsenzyme sind z.B. solche, die Fragmente mit glatten Enden liefern, wie z.B. Smal, Hpal und EcoRV, oder aber Enzyme, die kohäsive Enden bilden, wie z.B. EcoRI, Sacl und BamHI.

Sowohl Fragmente mit glatten Enden wie auch solche mit kohäsiven Enden, die einander komplementär sind, können mit Hilfe geeigneter DNA-Ligasen wieder zu einem einheitlichen durchgehenden DNA-Molekül verknüpft werden.

Glatte Enden können auch durch Behandlung von DNA-Fragmenten, die überhängende kohäsive Enden aufweisen, mit dem Klenow-Fragment der E.coli DNA-Polymerase durch Auffüllen der Lücken mit den entsprechenden komplementären Nukleotiden hergestellt werden.

Anderseits lassen sich auch kohäsive Enden künstlich herstellen, z.B. durch Anfügen komplementärer homopolymerer Schwänze an die Enden einer gewünschten DNA-Sequenz sowie des geschnittenen Vektormoleküls unter Verwendung einer terminalen Desoxynukleotidyl-Transferase oder aber durch Anfügen synthetischer Oligonukleotid-Sequenzen (Linker), die eine Restriktionsschnittstelle tragen und anschliessendes Schneiden mit dem entsprechenden Enzym.

Der Klonierungsvektor und die mit diesem Vektor transformierte Wirtszelle werden in der Regel dazu verwendet, die Kopienzahl des Vektors zu erhöhen. Mit einer erhöhten Kopienzahl ist es möglich, den Vektor, der die hybride Genkonstruktion trägt, zu isolieren und z.B. für die Einschleusung der chimären Gensequenz in die pflanzliche Zelle zu verwenden.

In einem weiteren Verfahrensschritt werden diese Plasmide dazu verwendet, die ein gewünschtes Genprodukt kodierenden Strukturgene oder nichtkodierende DNA-Sequenzen mit regulatorischer Funktion in die pflanzliche Zelle einzuschleusen und in das Pflanzengenom zu integrieren.

Ebenfalls umfasst vom breiten Konzept dieser Erfindung sind transgene Pflanzen, die mit Hilfe des zuvor beschriebenen erfindungsgemässen Verfahrens transformiert worden sind, d.h. die das eingeschleuste genetische Material nachweisbar enthalten und exprimieren sowie deren asexuelle und oder sexuelle Nachkommenschaft, welche noch das eingeschleuste genetische Material enthalten und somit in der Regel die durch die Transformation der Mutterpflanze bedingte(n) neue(n) und wünschenswerte(n) Eigenschaft(en) aufweisen, sowie allgemein pflanzliches Vermehrungsgut.

Unter den Begriff der asexuellen und/oder sexuellen Nachkommenschaft transgener Pflanzen fallen definitionsgemäss im Rahmen dieser Erfindung somit auch alle Mutanten und Varianten, welche noch das eingeschleuste genetische Material enthalten und somit in der Regel die charakteristischen Eigenschaften der transformierten Ausgangspflanze aufweisen, sowie sämtliche Kreuzungs- und Fusionsprodukte mit dem transformierten Pflanzenmaterial.

Unter dem Begriff des pflanzlichen Vermehrungsgutes sollen im Rahmen dieser Erfindung definitionsgemäss z.B. pflanzliche Protoplasten, Zellen, Zellklone, Zellagglomerate, Kallus-, Gewebe- und oder Organkulturen sowie Samen, Pollen, Eizellen, Zygoten, Embryonen oder anderes aus Keimbahnzellen hervorgegangenes Vermehrungsgut verstanden werden, wobei diese beispielhafte Aufzählung keine Limitierung des Erfindungsgegenstandes bedeutet.

Pflanzenteile, wie z.B. Blüten, Stengel, Früchte, Blätter, Wurzeln, die von transgenen Pflanzen oder deren Nachkommen stammen, die zuvor mit Hilfe des erfindungsgemässen Verfahrens transformiert worden sind und die somit wenigstens zum Teil aus transgenen Zellen aufgebaut sind, sind ebenfalls Gegenstand dieser Erfindung.

Das erfindungsgemässe Verfahren eignet sich für die Transformation aller Pflanzen, insbesondere solchen der systematischen Gruppen Angiospermae und Gymnospermae.

Unter den Gymnospermae sind von besonderem Interesse die Pflanzen der Klasse Coniferae.

Unter den Angiospermae sind neben den Laubbäumen und Sträuchern von besonderem Interesse Pflanzen der Familien Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae, Malvaceae oder Gramineae und der Ordnung Leguminosae und hier vor allem der Familie Papilionaceae. Bevorzugt sind Vertreter der Solanaceae, Cruciferae, Leguminosae, Malvaceae und Gramineae.

Zu den Zielkulturen im Rahmen der vorliegenden Erfindung zählen beispielsweise auch jene, ausgewählt aus der Reihe: Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum und Sorghum, Gossypium, Ipomoea, Passiflora, Cyclamen, Malus, Prunus, Rosa, Rubus, Populus, Santalum, Allium, Lilium, Narcissus, Ananas, Arachis, Phaseolus und Pisum.

Besonders bevozugt sind dabei Vertreter aus der Familie der Gramineae, wie z.B. Pflanzen, die grossflächig angebaut werden und hohe Ernteerträge liefern. Als Beispiele seien genannt: Mais, Reis, Weizen, Gerste, Roggen, Hafer, Hirse oder Sorghum sowie Weidegräser.

Weitere Zielkulturen, die besonders bevorzugt sind für die Verwendung in dem erfindungsgemässen Verfahren, sind beispielsweise Pflanzen der Gattungen Allium, Avena, Hordeum, Oryza, Panicum, Saccharum, Secale, Setaria, Sorghúm, Triticum, Zea, Musa, Cocos, Phoenix und Elaeis.

Der Nachweis einer erfolgreichen Transformation kann auf an sich bekannte Weise erfolgen, beispielsweise durch molekularbiologische Untersuchungen, zu denen besonders die "Southern blot"-Analyse gehört.

Die extrahierte DNA wird dabei zunächst mit Restriktionsenzymen behandelt, anschliessend einer Elektrophorese in einem 0,8%igen bis 1%igen Agarose-Gel unterworfen, auf eine Nitrozellulosemembran [Southern, E.M., J.Mol.Biol 98, 503-517(1975)] transferiert und mit der nachzuweisenden DNA, welche zuvor einer Nick Translation [Rigby, W.J., Dieckmann, M., Rhodes, C. und P. Berg, J.Mol.Biol. 113,237-251)] unterworfen wurde (DNA-spezifische Aktivitäten von $5 \times 10^8$ bis $10 \times 10^8$ c.p.m./μg), hybridisiert. Die Filter werden dreimal je eine Stunde lang mit einer wässrigen Lösung von 0.03 M Natriumcitrat und 0.3 M Natriumchlorid bei 65°C gewaschen. Die hybridisierte DNA wird durch Schwärzung eines Röntgenfilms während 24 bis 48 Stunden sichtbar gemacht.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn, es wird speziell darauf hingewiesen. Das gleiche gilt auch für alle beispielhaften Aufzählungen, die in der vorangegangenen Beschreibung enthalten sind.

Nichtlimitierende Ausführungsbeispiele:

Beispiel 1: Konstruktion des Plasmids pABDI

Man zerschneidet die frei zugänglichen Plasmide pkm 21 und pkm 244 [Beck, E., et al., Gene 19, 327-336(1982)] mit der Restriktions-Endonuclease PstI. Die Fragmente der Plasmide, welche für die Rekombination verwendet werden, werden durch Elektrophorese in 0,8%igem Agarose-Gel gereinigt. Das aus der Verbindung der Bruchstücke erhaltene Plasmid pkm 21244 enthält eine Kombination der 5'- und 3'-Bal 31 - Deletionen des NPT-II-Gens, wie von Beck et al. in Gene 19, 327-336 (1982) beschrieben. Um das Promotorsignal des Cauliflower-Mosaik-Virus mit dem HindIII-Fragment des Plasmids pkm 21244 zu verbinden, wird das Kupplungsplasmid pJPAX konstruiert. Das Kupplungsplasmid pJPAX wird aus den Plasmiden pUC8 und pUC9 [Messing, J. and J. Vieira, Gene 19, 269-276(1982)] erhalten. 10 Basenpaare von der Kupplungssequenz des Plasmids pUC9 werden durch Zerschneiden bei der HindIII- und der SalI-Position und nachfolgendem Auffüllen der haftfähigen Enden mit dem Polymerase-I-Klenow-Fragment [Jacobsen, H., et al., Eur. J. Biochem 45, 623 (1974)] und Verbinden der Polynukleotidkette, wodurch die HindIII-Position wieder hergestellt wird, herausgetrennt. Ein synthetisches Kupplungsglied von 8 Basenpaaren (XhoI) wird an der SmaI-Position dieser abgetrennten Kupplungssequenz eingefügt. Die Rekombination der geeigneten XorI- und HindIII-Fragmente des Plasmids pUC8 und des modifizierten Plasmids pUC9 ergibt das Plasmid pJPAX mit einer teilweise asymmetrischen Kupplungssequenz mit den aufeinander folgenden Restriktionsstellen: EcoRI, SmaI, BamHI, SalI, PstI, HindIII, BamHI, XhoI und EcoRI. Die CaMV Gen VI Promotor-Region, die mit dem NPT II Strukturgen verknüpft wird, stammt aus dem Genom des CaMV-Stammes CM 4-184, einer Variante des CaMV-Stammes CM 1841, dessen komplette Nukleotidsequenz bei Gardner RC et al, 1981 beschrieben ist. Die CaMV Promotor-Region wird in dem 6 kBp (kBp: 1000 Basenpaare) umfassenden Cosmid pHC 79, einem Abkömmling des E. coli Plasmids pBR322 [Hohn B and Collins J, 1980], kloniert, wobei das CaMV Genom sowie das Cosmid pHC79 mit BstII geschnitten und die resultierenden Bruchstücke miteinander verknüpft werden. Die Verbindung des 5'-Expressionssignals des Cauliflower-Mosaik-Virus-Gens VI und des HindIII-Fragments des NPT-II-Gens wird beim Plasmid pJPAX durch Einfügung der Promotorregion des Cauliflower-Mosaik-Virus-Gens VI zwischen die PstI-und die Hind III-Position bewirkt. Das so erhaltene Plasmid wird an der einzigen HindIII-Position aufgeschnitten und das HindIII-Fragment des Plasmids pkm 21244 wird in diese Schnittstelle in beiden Orientierungsrichtungen eingebaut, wobei man die Plasmide pJPAX Cakm + und pJPAX Cakm- erhält. Um eine EcoRV-Sequenz in der Nähe des 3'Terminationssignals des NPT-II-Hybridgens zu schaffen, wird ein BamHI-Fragment des Plasmids pJPAX Cakm + in die BamHI-Position des Plasmids pBR327 [Soberon, X. et al., Gene 9, 287-305 (1980)] eingebaut. Man erhält das Plasmid pBR327 Cakm. Das EcoRV-Fragment des Plasmids pBR327 Cakm, das die neue DNA-Konstruktion enthält, wird verwendet, um die EcoRV-Region des Cauliflower-Mosaik-Virus-Gens VI zu ersetzen, welches an der Sall-Position im Plasmid pUC8 kloniert ist, wodurch man die Proteinkodierende DNA-Sequenz des NPT-II-Gens unter die Kontrolle der 5′- und 3′- Expressionssignale des Cauliflower-Mosaik-Virus-Gens VI stellt. Die so hergestellten Plasmide tragen die Bezeichnung pABDI und pABDII.


Beispiel 2: Konstruktion des Plasmids pDH51

Das Plasmid pDH 51 ist bei Pietrzak et al., 1986 beschrieben. Dieses Plasmid enthält die 35S Promotor-Region und Terminator-Region von CaMV, die getrennt sind durch eine eingeschobene Polylinker-Region, die zahlreiche Klonierungsstellen enthält. Als Ausgangsmaterial für die 35S Promotor-Terminator Region dient ein ScaI-Fragment von CaMV "S", das die Nukleotide 6808-7632 der Genkarte (Frank G et al., Cell, 21: 285-294, 1980) umfasst, und das in die SmaI-Stelle von pUC18 (Norrander J. et al., Gene, 26: 101-106, 1983) eingespleisst wird, unter Bildung von Plasmid pDB21. Dies wird anschliessend mit dem Restriktionsenzym HphI verdaut. Die kohäsiven Enden werden durch Behandlung mit T4 DNA Polymerase entfernt.

Ein Fragment, welches die 35S Promotor-Region enthält [pUC18 pos. 21 (HphI)-412 (SmaI) plus CaMV pos. 6808-7437 (HphI)] wird mit KpnI Linkern verknüpft, mit NcoI (pos. 6909 CaMV) geschnitten, die kohäsiven Enden mit Hilfe des Klenow-Fragments der DNA-Polymerase aufgefüllt, mit EcoRI-Linkern verknüpft und schliesslich mit EcoRI und KpnI verdaut.

Das resultierende EcoRI KpnI-Fragment, das die CaMV Promotor-Sequenz enthält, wird dann in das Plasmid pUC18 zwischen die KpnI und EcoRI Restriktionsschnittstellen eingespleisst. Man erhält auf diese Weise das Plasmid pDG2.

Ein zweites Fragment, das die Terminationssignale trägt [CaMV pos. 7439 (HphI)-7632 plus pUC18 pos. 413-1550 (HphI)], wird mit EcoRI geschnitten, die kohäsiven Enden werden mit Klenow aufgefüllt, mit HindIII Linkern versehen und mit HindIII und SphI verdaut.

Das resultierende SphI-Hind111 Fragment, das die CaMV Terminator-Sequenz enthält, wird dann in das Plasmid pDG2 eingespleisst und zwar in die HindIII und SphI Restriktionsschnittstellen.


Beispiel 3: Konstruktion des Plasmids pHP23

Die Konstruktion von Plasmid pHP23 erfolgt durch Insertion eines EcoRV-Fragments des Plasmids pABD1, welches das APH(3′)II-Strukturgen sowie einen Teil der 19S RNA Promotor-Sequenz von CaMV enthält, in die SmaI-Schnittstelle des Plasmids pDH51.


Beispiel 4: Herstellung und Mikroinjektion von Tabakkeimlingen


4.1. Pflanzenmaterial

Samen von Nicotiana tabacum c.v. Petite Havanna SRI Pflanzen werden auf nicht steriler Erde gekeimt und zu Keimlingen herangezogen. Die Beleuchtung erfolgt einseitig von oben, während die Seiten und der Boden durch Anbringen einer schwarzen Beschichtung vor einer Bestrahlung geschützt werden. Dies führt dazu, dass die Sprossachse kurz gehalten wird. Die Keimungszeit hängt von der Keimaktivität der verwendeten Samen ab. Sie beträgt in der Regel zwischen 3 bis 6 Tagen.


4.2. Vorbereitung der Keimlinge für die Mikroinjektion

Wenn die Keimblätter eine Grösse von ca. 1,5 mm erreicht haben, werden die Keimlinge aus der Erde entnommen und die Vegetationskegel frei präpariert. Dabei wird entweder eines oder aber beide Keimblätter entfernt oder aber die Keimblätter werden mit Hilfe einer speziellen Haltevorrichtung soweit auseinandergezogen, bis das apikale Meristem sichtbar wird.

### 4.3 Mikroinjektion von pHP23 Plasmid-DNA in Tabakkeimlinge

#### 4.3.1. Apparatur

Die Mikroinjektion in den Sprossvegetationskegel von Tabakkeimlingen wird unter mikroskopischer Beobachtung an einem inversen Mikroskop oder einem Stereomikroskop mit einer Kombination aus Auf- und Durchlicht durchgeführt. Als Beleuchtung dient eine Kaltlichtquelle um eine Wärmeentwicklung zu vermeiden. Die Vergrösserung hängt von der Grösse der Keimlinge ab und schwankt zwischen 10-300facher Vergrösserung.

Die Manipulation der Keimlinge wird mit Hilfe von Mikromanipulatoren durchgeführt, die am Mikroskoptisch befestigt sein können (motorisch betriebene Manipulatoren) oder seitlich neben dem Mikroskop stehen (mechanische Manipulatoren). Die Mikromanipulatoren sind mit Kapillarhalterungen ausgerüstet.

Als Mikroinjektionskapillare werden Mikroelektrodenkapillaren mit eingeschmolzenen Glasfilamenten aus Borosilikat verwendet, die einen Aussendurchmesser von 1,00 mm bis 1,50 mm aufweisen. Zur Fixierung der Keimlinge beim Injektionsvorgang werden speziell für diesen Zweck hergestellte Klemmvorrichtungen verwendet. Auf dieser Klemmvorrichtung werden die Keimblätter auf einer speziellen Unterlage, z.B. einer Metallhalterung, festgeklemmt und somit fixiert; diese Unterlagen sind gegeneinander beweglich, sodass die Keimblätter soweit auseinandergezogen werden können, bis die Sicht auf den Vegetationskegel freigeben und dieser somit für die sich anschliessende Mikroinjektion zugänglich wird. Die Injektionskapillare wird auf einem handelsüblichen Kapillaren-Ziehgerät ausgezogen, sodass geeignete Injektionskapillaren (Spitzdurchmesser ca. $\leq$ 1 $\mu$m) entstehen. Selbstverständlich kann auch jedes andere geeignete Verfahren zur Freilegung des Vegetationskegels angewendet werden.

Die Injektion der DNA-Lösung erfolgt über einen Mikroinjektor. Es handelt sich dabei um eine pneumatisch betriebene Druckvorrichtung, die eine sehr fein dosierte Injektion in die Zellen des Vegetationskegels ermöglicht. Das Injektionsvolumen schwankt je nach Zellgrösse zwischen 1 pl und 100 pl. Geeignete Mikroinjektoren können beispielsweise bei der Firma Eppendorf, FRG oder Fa. Bachhofer, FRG bezogen werden.

#### 4.3.2. DNA-Lösung

Bei der mikroinjizierten DNA-Lösung handelt es sich um überspiralisierte oder linearisierte pHP23 DNA oder aber um ein Gemisch aus überspiralisierter und linearisierter pHP23 DNA in einem Tris-HCl Puffer, bestehend aus 50 mM Tris-HCl, pH 7-7,8 und 50 mM NaCl.

#### 4.3.3. Mikroinjektion

Die Mikroinjektion der zuvor beschriebenen DNA-haltigen Lösung in die Zellen des Apikalmeristems von Tabakkeimlingen erfolgt analog dem bei Neuhaus et al., 1984; 1986 beschriebenen Verfahren.

Die Keimlinge werden zunächst mit Hilfe beider oder aber auch nur eines Keimblattes auf jeweils einer Metallhalterung befestigt. Da diese Metallhalterungen gegeneinander beweglich sind, lassen sich die Keimblätter (das Keimblatt) vorsichtig auseinanderziehen, bis das Apikalmeristem soweit freigelegt ist, dass die sich anschliessende Mikroinjektion ohne Schwierigkeiten durchgeführt werden kann.

Jeder Keimling wird je nach Grösse des freigelegten Apikalmeristems 4x bis 60x injiziert und zwischen den einzelnen Injektionsvorgängen gedreht, sodass möglichst viele Zellen getroffen werden. Anschliessend werden die injizierten Keimlinge in Erde zurücküberführt und weiterkultiviert.

### 4.4. Kultivierung der mikroinjizierten Tabakkeimlinge und Heranziehen ganzer Tabak-Pflanzen

Nach der Mikroinjektion werden die Keimlinge in Plastikdosen mit einem Durchmesser von 9 cm und einer Höhe von 5 cm auf Erde zurücküberführt und dort bei 25° bis 28°C und einem Hell/Dunkel Rhythmus von 16:8 Stunden (4000 Lux, kaltes Weisslicht), bei einer Luftfeuchtigkeit von 70 % bis 80 % und normalem Druck (Luftdruck, ca. 760 mbar) kultiviert, bis die Pflänzchen eine Grösse von 3 cm erreicht haben. Dies geschieht in der Regel nach 2 bis 5 Tagen; Danach werden die Pflanzen in das Gewächshaus gebracht, in Erde überführt und wie normale Tabakkeimlinge bis zur Reife der Pflanzen herangezogen, weiterkultiviert.

Bei den auf die zuvor beschriebene Weise erhaltenen Pflanzen handelt es sich um heterogene Transformanten, deren chimäre Natur mit Hilfe der unter Abschnitt 7 und 8 beschriebenen Verfahren untersucht werden kann.

Beispiel 5: Herstellung und Mikroinjektion von isolierten Apikalmeristemen von Tabakkeimlingen

5.1. Pflanzenmaterial

Samen von Nicotiana tabacum c.v. Petite Havanna SR1 Pflanzen werden Oberflächensterilisiert. Zu diesem Zweck werden die Pflanzen zunächst kurz (für wenige Sekunden) in 70 % Ethanol gewaschen und anschliessend sofort mit sterilem destilliertem Wasser gespült. Es folgt eine 30 minütige Behandlung in einer 1.5 % Calciumhypochloritlösung. Danach werden die Pflanzen dreimal in zuvor sterilisiertem destilliertem Wasser gewaschen und getrocknet. Anschliessend werden etwa 50 Samen in eine Petrischale (10 cm Durchmesser) mit einem gängigen Nährmedium (T1) gelegt und zur Keimung gebracht. Die Beleuchtung erfolgt einseitig von oben, während die Seiten und der Boden durch Anbringen einer schwarzen Beschichtung vor Bestrahlung geschützt sind. Diese Massnahmen führen dazu, dass die Sprossachsen der Keimlinge kurz gehalten werden, was für die sich anschliessende Mikroinjektion vorteilhaft ist.

5.2. Mikroinjektion von pHP23 Plasmid DNA in Tabakkeimlinge

Die Vorbereitung der Keimlinge für die Mikroinjektion sowie die Mikroinjektion selbst werden analog zu den in den Abschnitten 4.2 und 4.3 beschriebenen Verfahren durchgeführt.

5.3. Isolierung des mikroinjizierten Vegetationskegels

Nach der Mikroinjektion werden den Keimlingen unter einem Stereomikroskop beide Keimblätter sowie weitere, eventuell noch vorhandene Blattansätze entfernt. Die freigelegte Sprossspitze wird ca. 0.5 mm bis 2 mm vorzugsweise aber 1 mm unterhalb vom übrigen Spross mit Hilfe eines Skalpells abgetrennt und auf einem geeigneten Kulturmedium (T2) weiterkultiviert. Ueblicherweise verwendet man für die Kultivierung der isolierten Sprossspitzen Terrasaki-Platten (Fa. Nunc, Dänemark), wobei diese auf 10 $\mu$l bis 20 $\mu$l T2 Medium kultiviert werden.

Nach ca. 5 bis 15 Tagen. vorzugsweise aber nach 10 Tagen, werden die sich entwickelnden Apikalmeristeme auf ein selektives Kulturmedium überführt (T3). Man verwendet dabei üblicherweise Costar-Platten ("Costar-wells"; 24 wells; 16 mm Durchmesser Fa. Tissue Culture Cluster, Cambridge, Mass.) mit einem T2 Medium, das als selektiven Zusatz das Antibiotikum Kanamycin in einer Konzentration von 20 $\mu$g/ml bis 200 $\mu$g/ml, vorzugsweise aber von 50 $\mu$g/ml enthält (T3). Unter diesen Selektionsbedingungen überleben nur diejenigen Zellen, die das mikroinjizierte Resistenzgen in ihr Genom eingebaut enthalten und dieses auch exprimieren, da nur diese Zellen gegen das Antibiotikum resistent sind. Nach einer weiteren Kultivierungszeit von 10 bis 20 Tagen, vorzugsweise von 15 Tagen, werden die wachsenden Zellverbände auf ein weiteres Kulturmedium übertragen (T4), welches ebenfalls das Antibiotikum Kanamycin in gleicher Konzentration enthält, welches aber die Zellverbände durch die Wahl seiner Hormonkonzentration zu einer vermehrten adventiven Sprossregeneration anregt. Auch in diesem Fall werden sich nur diejenigen Sprosse entwickeln, die das injizierte Resistenzgen in ihr Genom integriert haben, da nach wie vor von Seiten des Mediums ein Selektionsdruck ausgeübt wird.

Abschliessend werden die regenerierten Sprosse durch Übertragung auf geeignete Medien (T5) bewurzelt, in Erde übertragen und schliesslich im Gewächshaus zur Samenreife herangezogen.

Beispiel 6: Nachweis des NPT-II-Gens im Pflanzenerbgut

Blattgewebe der regenerierten und transformierten Pflanzen werden bei 0° C in 15 %-iger Saccharoselösung, enthaltend 50 mMol/l Ethylendiamin-N,N,N',N'-tetraessigsäure, EDTA), 0,25 Mol/l Natriumchlorid und 50 mMol/l $\alpha,\alpha,\alpha$-Tris-(hydroxymethyl)methylamin-Hydrochlorid (TRIS-HCl) bei pH 8.0 homogenisiert.

Durch Zentrifugieren des Homogenisats für 5 Minuten bei 1000 g trennt man grob die Zellkerne ab. Die Zellkerne werden in 15 %-iger Saccharoselösung, enthaltend 50 mMol/l EDTA und 50 mMol/l TRIS-HCl, bei pH 8,0 resuspendiert, mit Natrium-dodecylsulfat (SDS) bis zu einer Endkonzentration von 0,2 % versetzt und 10 Minuten auf 70° C erhitzt. Nach Abkühlung auf 20-25° C wird der Mischung Kaliumacetat bis zu einer Konzentration von 0,5 Mol/l zugesetzt. Dieses Gemisch wird 1 Stunde bei 0° C inkubiert. Der ausgefallene Niederschlag wird durch Zentrifugieren (15 Minuten bei 4° C in einer Mikrozentrifuge) zentrifugiert. Aus der überstehenden Flüssigkeit wird durch Versetzen mit dem 2,5-fachen Volumen Ethanol bei 20-25° C die DNA ausgefällt. Die abgetrennte DNA wird in einer Lösung von 10 mM TRIS-HCl, enthaltend 10 μg/ml Ribonuclease A, gelöst, 10 Minuten bei 37° C inkubiert, mit Proteinase K bis zu einer Konzentration von 250 μg/ml versetzt und eine weitere Stunde bei 37° C inkubiert. Die Proteinase K wird durch Phenol- und Chloroform/Isoamylalkohol-Extraktionen entfernt. Aus der wässrigen Phase wird die DNA durch Zusatz von 0,6 Volumenteilen 0,6M isopropanolischer Natriumacetat-Lösung ausgefällt und in 50 μl einer Lösung, enthaltend 10 mMol/l TRIS-HCl und 5 mMol/l EDTA, bei pH 7,5 gelöst. Durch diese Präparation erhält man DNA-Sequenzen, die vorwiegend mehr als 50'000 Basenpaare enthalten. Restriktion dieser DNA mit EcoRV-Endonuclease, Hybridisierung der Fragmente mit radioaktiv markierten HindIII-Bruchstücken des NPT-II-Gens und Vergleich mit dem Plasmid pABDI zeigt in einer "Southern Blot"-Analyse die Anwesenheit des NPT-II-Gens in der Zellkern-DNA der transformierten Pflanzen-Zellen.

Beispiel 7: "Dot-blot"-Analyse

10 μg genomischer DNA werden über Nacht mit EcoRI verdaut und anschliessend ohne vorherige elektrophoretische Auftrennung direkt punktförmig auf eine Nitrozellulosemembran aufgetragen (vgl. Verfahrensvorschrift von Schleicher und Schüll GmbH, Dassel, FRG).

Die Hybridisierungsreaktion und die anschliessende Autoradiographie werden in analoger Weise zu dem Southern blot-Verfahren durchgeführt.

Beispiel 8: Southern blot-Analyse:

Die Southern blot-Analyse der Pflanzen DNA wird entsprechend einer bei Paszkowski und Saul, 1983, beschriebenen Methode durchgeführt.

Ungefähr 5 μg genomischer DNA werden wahlweise mit EcoRV oder HindIII-Restriktionsenzymen geschnitten und die erhaltenen Bruchstücke durch Elektrophorese in einem 0,8 % bis 1 % Standard-Agarose-Gel aufgetrennt. (Maniatis T et al., 1982).

Die Nick-Translation wird dabei ersetzt durch die "random primer" Methode, die bei Feinberg und Vogelstein, 1983, beschrieben ist.

Beispiel 9: Untersuchung der Aminoglykosidphosphotransferase-Enzymaktivität transformierter Pflanzen

Blattstücke (100 bis 200 mg) werden in 20 μl Extraktionspuffer in einem Eppendorf-Zentrifugenröhrchen homogenisiert. Der Puffer ist eine Modifikation des von Herrera-Estrella, L., DeBlock, M., Messens, E., Hernalsteens, J.-P., Van Montagu, M. und J. Schell, EMBO J. 2, 987-995 (1983) verwendeten Puffers, in welchem Albumin aus Rinderblut durch 0,1 M Saccharose ersetzt wird. Die Extrakte werden 5 Minuten lang bei 12'000 g zentrifugiert und die überstehende Phase mit Bromphenolblau bis zu einer Endkonzentration von 0,004 % versetzt. Durch Elektrophorese in einem 10%igen, nicht denaturierenden Polyacrylamid-Gel werden die Proteine aus 35 μl der überstehenden Phase separiert Das Gel wird mit einem Kanamycin und γ$^{32}$P-markierte ATP enthaltenden Agarose-Gel überschichtet, inkubiert, und die phosphorylierten Reaktionsprodukte werden auf Whatman-p81-Phosphozellulose-Papier übertragen. Das Papier wird sechsmal mit deionisiertem Wasser von 90° C gewaschen und dann autoradiografiert.

Ergebnisse:

Die Regeneration ganzer, transgener Pflanzen aus mikroinjizierten Apikalmeristemen via direkter Aufzucht von in vitro Kulturpflanzen unter Selektionsdruck konnte beim Tabak durchgeführt werden, wobei die erzielten Regenerationsraten sehr hoch liegen. Bei in vitro Kulturen mit Selektion werden die Regeneranten zur Bestimmung der Transformations-(Expressions-)frequenz verwendet.

Für alle übrigen Pflanzen, die direkt aus den mikroinjizierten Keimlingen erhältlich sind, wird die folgende Methode angewendet: nach Selbstbefruchtung werden die Samen dieser Pflanzen zum Keimen gebracht und diese Pflanzengeneration wird dazu verwendet, um den Nachweis für die Transformation und Expression des Gens zu erbringen. Noch 6 Wochen nach der erfolgten Mikroinjektion konnte das injizierte NPTII-Gen anhand seiner Expression nachgewiesen werden. Die Expression des integrierten Gens wird mit Hilfe des unter Beispiel 9 (Untersuchung der Aminoglykosidphosphotransferase-Enzymaktivität) beschriebenen Enzym-Assays nachgewiesen. Die auf diese Weise ermittelten Expressionsfrequenzen liegen in einem Bereich zwischen 5% und 35%. Eine stabile Integration der vollständigen mikroinjizierten Gene in die hochmolekulare DNA der Fl-Generation, hervorgegangen aus dem primären Regeneranten, kann mit Hilfe der Southern blot-Analyse der genomischen DNA besagter Fl-Generationspflanzen demonstriert werden.

Hinterlegung:

Das im Rahmen der vorliegenden Erfindung verwendete Plasmid pHP23 wurde bei der als Internationale Hinterlegungsstelle anerkannten 'Deutschen Sammlung von Mikroorganismen' (DSM), in Braunschweig, Bundesrepublik Deutschland, entsprechend den Anforderungen des Budapester Vertrages für die Internationale Anerkennung der Hinterlegung von Mikroorganismen zum Zwecke der Patentierung, hinterlegt. Eine Erklärung zur Lebensfähigkeit der hinterlegten Proben wurde durch die besagte Internationale Hinterlegungsstelle ausgestellt.

EP 0 400 553 A1

| Mikroorganismus | Hinterlegungsdatum | Hinterlegungs-Nummer* | Datum der Lebensfähigkeits-Bescheinigung |
|---|---|---|---|
| pHP23 (Escherichia coli DH1 transformiert mit pHP23 Plasmid DNA | 21.12.1987 | DSM 4323 | 21.12.1987 |

*ausgegeben durch die oben bezeichnete Internationale Hinterlegungsstelle.

Medien

| mg/l | T1 | T2 | T3 | T4 | T5 |
|---|---|---|---|---|---|
| $Ca(NO_3)_2$ $(4H_2O)$ | 1000 | - | - | - | - |
| $KNO_3$ | 250 | 1010 | 1010 | 1010 | 1900 |
| $KH_2PO_4$ | 250 | 136 | 136 | 136 | 170 |
| $NH_4NO_3$ | - | 800 | 800 | 800 | 1650 |
| $CaCl_2 \cdot 2H_2O$ | - | 440 | 440 | 440 | 440 |
| $MgSO_4 \cdot 7H_2O$ | 250 | 740 | 740 | 740 | 370 |
| $NH_4 \cdot$Succinate | - | 50 | 50 | 50 | - |
| $Na_2EDTA$ | - | 74,6 | 74,6 | 74,6 | 74,6 |
| $FeCl_3 \cdot 6H_2O$ | - | 27,0 | 27,0 | 27,0 | 27,0 |
| Fe-EDTA | 6,4 | - | - | - | - |
| $H_3BO_3$ | - | 3 | 3 | 3 | 6,2 |
| KI | - | 0,75 | 0,75 | 0,75 | 0,83 |
| $MnSO_4 \cdot H_2O$ | - | 10 | 10 | 10 | 16,9 |
| $ZnSO_4 \cdot 7H_2O$ | - | 2 | 2 | 2 | 8,6 |
| $CuSO_4 \cdot 5H_2O$ | - | 0,025 | 0,025 | 0,025 | 0,025 |
| $Na_2MoO_4 \cdot 2H_2O$ | - | 0,25 | 0,25 | 0,25 | 0,25 |
| $CoCl_2 \cdot 6H_2O$ | - | 0,025 | 0,025 | 0,025 | - |
| $CoSO_4 \cdot 7H_2O$ | - | - | - | - | 0,03 |
| m-Inosit | - | 100 | 100 | 100 | 100 |
| Pyridoxin·HCl | - | 1 | 1 | 1 | - |
| Thiamin·HCl | - | 10 | 10 | 10 | 0,04 |
| Nicotinsäure | - | 1 | 1 | 1 | - |
| Saccharose | - | 30 g/l | 30 g/l | 20 g/l | 30 g/l |
| Mannit | - | 30 g/l | - | - | - |

| mg/l | T1 | T2 | T3 | T4 | T5 |
|---|---|---|---|---|---|
| Agar | 8000 | - | 6000 | 6000 | 6000 |
| Sea Plaque® Agarose | - | 6000 | - | - | - |
| NAA (Naphthyl-1-essigsäure) | - | 0,1 | 0,1 | - | 0,1 |
| BAP (Benzylamino-purin) | - | 1 | 1 | 0,25 | - |
| IBA (3-Indolbutter-säure) | - | - | - | - | 1 |

Literaturverzeichnis

1. Barton CA et al, Plant Physiol., 85: 1103-1109,1987

2. Beck et al., Gene. 19: 327-336, 1982

3. Berry-Lowe et al., J. Mol., Appl. Genet., 1: 483-498, 1982

4. Birky et al., Biochim. Biophys. Acta, 67: 326-328, 1963

5. Cashmore A, "Genetic Engineering of Plants, an Agricultural Perspective", Plenum, New York, 1983, Seite 29-38

6. Chilton M-D, Scientific American, 248: 50-59, 1983

7. Coruzzi G et al, The Journal of Biological Chemistry, 258: 1399, 1983

8. De La Pena et al, Nature, 325:274-276,1987

9. Dunsmuir P et al, J. Mol. Appl. Genet., 2: 285, 1983

10. Feinberg und Vogelstein, Analytical Biochemistry, 132: 6-13, 1983

11. Frank G et al, Cell, 21: 285-294, 1980

12. Gardner RC et al, Nucl. Acids Res., 9: 2871-2888, 1981

13. Grimsley NH et al, Nature, 325: 177-179,1987

14. Hammond et al, J Biol. Chem., 259: 9883-90, 1984

15. Harris R. et al, Plant Cell Reports, 7: 337-340, 1988

16. Hernalsteens JP et al, EMBO J., 3:3039-3041,1984

17. Herrera-Estrella L et al, EMBO J., 2: 987-995, 1983

18. Hilder VA et al, Nature, 330: 160-163, 1987

19. Hohn B und Collins J, Gene, 11: 291-298, 1980

20. Hohn T et al, "Molecular Biology of Plant Tumors", Academic Press, Seite 549-560, 1982

21. Hooykaas-Van-Slogteren et al, Nature 311: 763-764, 1984

22. Howard et al, Planta, 170: 535, 1977

23. Hakura K et al, J Am. Chem. Soc., 97: 7327, 1975

24. Jacobson H et al, Eur. J. Biochem., 45: 623, 1974

25. Maniatis et al, Molecular Cloning, Cold Spring Harbor Laboratories, 1982

26. Messing und Vieira, Gene 19: 269-276, 1982

27. Morelli et al, Nature, 315: 200, 1985

28. Morikawa H und Yamada Y, Plant Cell Physiol., 26: 229-236, 1985

29. Neuhaus G et al, EMBO J, 3: 2169-2172, 1984

30. Neuhaus G et al, EMBO J, 5: 1437-1444, 1986

31. Neuhaus G et al, Theor. Appl. Genet., 75: 30-36, 1987

32. Nomura K und Komamine A, Plant Sci., 44: 53-58, 1986

33. Norrander J et al, Gene, 26: 101-106, 1983

34. Odell JT et al, Nature, 313: 810, 1985

**EP 0 400 553 A1**

35. Paszkowski J et al, EMBO J, 3: 2717-2722, 1984

36. Paszkowski J und Saul M, Methods in Enzymology, 118: 627-646, 1986

37. Paszkowski und Saul, "Direct Gene Transfer to Plants" in: Methods for Plant Molecular Biology, eds. A & H Weisbach. Academic Press, 1988, pages 447-463

38. Pennica P et al, Nature, 301: 214, 1983

39. Pietrzak M et al, Nucl. Acids Res., 14: 5868, 1986

40. Poehlman JM, "Breeding Field Crops, Third Edition, ,AVI Publishing Company. Inc., Westport, Connecticut, 1987"

41. Potrykus I et al, "Direct Gene Transfer to Protoplasts: An Efficient and Generally Applicable Method for Stable Alterations of Plant Genoms" in: Freeling M (ed.), Plant Genetics, A.R. Liss Inc., New York, pp. 181-199, 1986

42. Rhodes CA et al. Biotechnology, 6: 56-60, 1988

43. Rigby WJ et al, J Mol. Biol., 113: 237-251, 1977

44. Rogers SG et al, "Gene Transfer in Plants: Production of Transformed Plants Using Ti-Plasmid Vectors, in: Methods for Plant Molecular Biology, eds. A & H Weisbach, Academic Press. 1988. pages 423-436

45. Ryan C et al, Ann Rev. Plant Physiol., 24: 173-196, 1973

46. Soberon X et al, Gene. 9: 287-305, 1980

47. Southern EM, J. Mol. Biol., 98: 503-517, 1975

48. Spangenberg G, "Manipulation individueller Zellen der Nutzpflanze Brassica napus L. mit Hilfe von Elektrofusion, Zellrekonstruktion und Mikroinjektion", PhD thesis, Universität Heidelberg, 1986

49. Spena et al. EMBO J, 4: 2736, 1985

50. Steinbiss HH und Stable P, Protoplasma, 116: 223-227, 1983

51. Toriyama K et al, Theor. Appl. Genet.,73: 16-19, 1986

52. Vaeck M et al. Nature, 328: 33-37, 1987

53. Wienand U et al, Mol. Gen. Genet., 182: 440-444, 1981

54. Yamada A et al, Plant Cell Rep., 5: 85-88, 1986

**Ansprüche**

1. Verfahren zur Einschleusung genetischen Materials in Pflanzen. dadurch gekennzeichnet. dass man eine DNA-haltige Lösung in die Zellen der zuvor freigelegten Apikal-und oder Axialmeristeme von Keimpflanzen mikroinjiziert und ausgehen von besagten mikroinjizierten Keimpflanzen oder von Teilen davon ganze Pflanzen heranzieht und nach Zwischenschaltung eines oder mehrerer sexueller oder asexueller Vermehrungsschritte die so erhältliche. homogen transformierte, asexuelle oder sexuelle Nachkommenschaft besagter heterogen transformierter Ausgangspflanzen mit Hilfe bekannter Verfahren selektioniert.

2. Verfahren gemäss Anspruch 1. dadurch gekennzeichnet, dass das sich bei besagten Pflanzen um Keimlinge handelt. die sich in einem Entwicklungsstadium befinden, das sich von der beginnenden Kotyledonarentwicklung bis hin zur Sekundärblattentwicklung erstreckt.

3. Verfahren gemäss Anspruch 1, das durch die folgenden spezifischen Verfahrensschritte gekennzeichnet ist:

a) Keimung von Samen der jeweiligen Zielpflanze und Selektion geeigneter Keimungsstadien;

b) Freilegen des Apikal- und oder Axialmeristems für die sich anschliessende Mikroinjektion;

c) Mikroinjektion einer DNA-haltigen Lösung enthaltend ein oder mehrere DNA-Fragmente in die Zellen des unter b) freigelegten Meristems;

d) Anzucht der mikroinjizierten Keimlinge zu vollständigen, heterogen transformierten Pflanzen;

e) Herstellung sexueller oder asexueller Nachkommenschaft der gemäss Punkt d) erhältlichen heterogen transformierten Pflanzen und Auslesen der homogen transformierten Nachkommen.

4. Verfahren gemäss Anspruch 1, das durch die folgenden spezifischen Verfahrensschritte gekennzeichnet ist:

a) Keimung von Samen der jeweiligen Zielpflanze und Selektion geeigneter Keimungsstadien;

b) Freilegen des Apikal- und oder Axialmeristems für die sich anschliessende Mikroinjektion;

c) Mikroinjektion einer DNA-haltigen Lösung enthaltend ein oder mehrere DNA-Fragmente in die Zellen des unter b) freigelegten Meristems;

d₁) Isolierung und in vitro Regeneration von mikroinjizierten Apikal- und oder Axialmeristemen ohne Selektionsdruck zu vollständigen, heterogen transformierten Pflanzen; oder

d₂) Isolierung und in vitro Regeneration von mikroinjizierten Apikal- und/oder Axialmeristemen unter

24

Selektionsdrück zu vollständigen, homogen transformierten Pflanzen;

e) Herstellung sexueller oder asexueller Nachkommenschaft der unter Punkt $d_1$) erhältlichen heterogen transformierten Pflanzen und Auslese der homogen transformierten Nachkommen.

5. Verfahren gemäss einem der Ansprüche 3 oder 4, dadurch gekennzeichnet, dass der Mikroinjektionsvorgang in folgenden Teilschritten abläuft:

a) Fixieren der Keimlinge mit Hilfe einer speziellen Haltevorrichtung

b) Injektion der DNA haltigen Lösung in die Zellen des Apikal- und/oder Axialmeristems mit Hilfe der Mikroinjektionskapillare

c) Drehen der Keimlinge und erneute Injektion.

6. Verfahren gemäss Anspruch 5, dadurch gekennzeichnet, dass es sich bei den Mikroinjektionskapillaren um Mikroelektroden mit eingeschmolzenen Glasfilamenten aus Borosilikat handelt.

7. Verfahren gemäss Anspruch 5, dadurch gekennzeichnet, dass man den Mikroinjektionsvorgang 1 bis 1000 mal pro Meristem durchführt.

8. Verfahren gemäss Anspruch 5, dadurch gekennzeichnet, dass das Injektionsvolumen 1 pl bis 100 pl pro injizierter Zelle beträgt.

9. Verfahren gemäss Anspruch 5 dadurch gekennzeichnet, dass die DNA-Konzentration der injizierten DNA-haltigen Lösung zwischen 0,1 $\mu$g/$\mu$l und 10 $\mu$g/$\mu$l beträgt.

10. Verfahren gemäss Anspruch 5, dadurch gekennzeichnet, dass besagte DNA-haltige Injektionslösung die transformierende DNA in linearisierter Form enthält.

11. Verfahren gemäss Anspruch 5, dadurch gekennzeichnet, dass besagte DNA-haltige Injektionslösung die transformierende DNA in überspiralisierter Form enthält.

12. Verfahren gemäss Anspruch 5, dadurch gekennzeichnet, dass besagte DNA-haltige Injektionslösung die transformierende DNA in Form eines Gemisches aus linearisierter und überspiralisierter DNA enthält.

13. Verfahren gemäss Anspruch 5, dadurch gekennzeichnet, dass besagtes DNA-haltige Injektionslösung ein rekombinantes DNA-Molekül enthält, das im wesentlichen aus einem Strukturgen besteht, das für eine nützliche und wünschenswerte Eigenschaft kodiert und das mit in der pflanzlichen Zelle aktiven Expressionssignalen in operabler Weise verknüpft ist.

14. Verfahren gemäss Anspruch 13, dadurch gekennzeichnet, dass besagtes Strukturgen der transformierten Pflanze eine erhöhte Toleranz oder Resistenz gegenüber Pathogenen, Herbiziden, Insektiziden oder anderen Bioziden verleiht.

15. Verfahren zur Herstellung reinerbiger homozygoter Transformanten gemäss Ansprüche 3, dadurch gekennzeichnet, dass man

a) heterogen transformierte Pflanzen, die einer Selbstbestäubung zugänglich sind, d.h. die keine Selbstinkompatibilitäten aufweisen, zur Herstellung von Inzuchtlinien wiederholt mit sich selbst kreuzt; oder

c) heterogen transformierte Pflanzen, die einer Selbstbestäubung nicht unmittelbar zugänglich sind, zur Herstellung von Inzuchtlinien wiederholt mit Pflanzen kreuzt, die einen hohen Verwandschaftsgrad aufweisen.

16. Verfahren gemäss Anspruch 3, dadurch gekennzeichnet, dass die Keimlinge nach erfolgter Mikroinjektion direkt in Erde übertragen und wie normale Sämlinge zu ganzen Pflanzen herangezogen werden.

17. Verfahren gemäss Anspruch 3, dadurch gekennzeichnet, dass die Keimlinge nach erfolgter Mikroinjektion auf ein spezifisches Kultivierungsmedium überführt werden, das eine für die weitere Entwicklung des Sämlings zur ganzen Pflanze geeignete Zusammensetzung aufweist.

18. Verfahren gemäss Anspruch 4, dadurch gekennzeichnet, dass man von Pflanzen, die einer Regeneration ausgehend von isolierten Meristemen zugänglich sind, die mikroinjizierten Apikal- und/oder Axialmeristeme entweder unmittelbar nach der erfolgten Mikroinjektion oder aber zu einem beliebigen späteren Zeitpunkt mit oder ohne Selektionsdruck isoliert und diese zu morphogenenen Kulturen heranzieht, aus denen dann wiederum in vitro mit Hilfe bekannter Verfahren ganze Pflanzen regeneriert werden können.

19. Verfahren gemäss Anspruch 18, dadurch gekennzeichnet, dass die Kultivierung der isolierten mikroinjizierten Apikal- und/oder Axialmeristeme in einem Kultivierungsmedium durchgeführt wird, das die Bildung adventiver Sprossknospen oder adentiver Embryonen anregt und das mit einem osmotisch neutralen Verdickungs- oder Geliermittel versetzt ist

20. Verfahren gemäss Anspruch 19, dadurch gekennzeichnet, dass es sich bei besagtem Verdickungsmittel um Co-Polymere aus Saccharose und Epichlorhydrin mit einem Molekulargewicht zwischen 70 000 und 400 000 handelt.

21. Verfahren gemäss Anspruch 19, dadurch gekennzeichnet, dass es sich bei besagtem Geliermittel um einen ausgewählt aus der Gruppe bestehend aus Agar, Agarose, Alginat, Pektinat, Gelrite® oder Gelatine handelt.

22. Verfahren gemäss Anspruch 19, dadurch gekennzeichnet, dass das Kulturmedium Phytohormone aus der Gruppe der Auxine und der Cytokinine in einer Konzentration von jeweils 0,1 mg/l bis 20,0 mg/l enthält, abhängig vom jeweiligen Entwicklungsstand der morphogenen Kultur.

23. Verfahren gemäss Anspruch 19, dadurch gekennzeichnet, dass der pH-Wert des Kulturmediums zwischen pH 5.2 und pH 6.5 beträgt.

24. Verfahren gemäss Anspruch 19, dadurch gekennzeichnet, dass die Kultivierungsdichte der Meristeme zwischen 1 Meristem/µl Kulturmedium und 50 Meristemen/µl Kulturmedium beträgt.

25. Verfahren gemäss Anspruch 19, dadurch gekennzeichnet, dass die Kultivierung der Meristeme zumindest zeitweise in einem Zweikompartimenten-System durchgeführt wird.

26. Verfahren gemäss Anspruch 4, dadurch gekennzeichnet, dass man die Meristemkulturen, wenn sie ein bestimmtes Entwicklungsstadium erreicht haben zur Differenzierung der Spross- und Wurzelscheitel auf feste Kulturmedien, die üblicherweise für die Regeneration von Pflanzen verwendet werden. überführt.

27. Verfahren gemäss Anspruch 4, dadurch gekennzeichnet, dass die Kulturmedien eine Selektivsubstanz enthalten, die eine positive Selektion der transgenen Zellen ermöglicht.

28. Verfahren gemäss Anspruch 4, dadurch gekennzeichnet, dass man die regenerierten Pflänzchen in Erde pflanzt und in gleicher Weise wie normale Sämlinge weiterbehandelt.

29. Verfahren zur Herstellung transgener Pflanzen gemäss Anspruch 1.

30. Transgene Pflanzen sowie deren sexuelle und asexuelle Nachkommenschaft. hergestellt gemäss einem Verfahren nach Anspruch 1, dadurch gekennzeichnet, dass ein Grossteil der somatischen Zellen und/oder der Keimzellen besagter Pflanzen sowie deren Nachkommen transformiert sind.

31. Transgenes Saatgut von Pflanzen gemäss Anspruch 30.

32. Transformierte lebensfähige Teile von Pflanzen gemäss einem der Ansprüche 30 und 31, ausgewählt aus der Gruppe bestehend aus Protoplasten. Zellen, Zellklonen, Zellagglomeraten, Kallus- und/oder Gewebekulturen, Samen. Pollen, Eizellen, Zygoten, Embryonen sowie die daraus abgeleiteten Nachkommen.

33. Alle Kreuzungs- und Fusionsprodukte mit dem in den Ansprüchen 30 bis 32 definierten transformierten Pflanzenmaterial, die noch das eingeschleuste genetische Material enthalten.

34. Varianten und Mutanten von transgenen Pflanzen bzw. von lebensfähigen Teilen davon sowie von deren sexueller und asexueller Nachkommenschaft gemäss einem der Ansprüche 30 bis 32, die noch das eingeschleuste genetische Material enthalten.

35. Pflanzenteile, wie z.B. Blüten. Stengel, Früchte, Blätter, Wurzeln, die von transgenen Pflanzen oder deren Nachkommen stammen, die zuvor mit Hilfe des erfindungsgemässen Verfahrens transformiert worden sind und die somit wenigstens zum Teil aus transgenen Zellen aufgebaut sind.

Patentansprüche für folgende Vertragsstaaten: GR, ES

1. Verfahren zur Einschleusung genetischen Materials in Pflanzen, dadurch gekennzeichnet, dass man eine DNA-haltige Lösung in die Zellen der zuvor freigelegten Apikal-und/oder Axialmeristeme von Keimpflanzen mikroinjiziert und ausgehen von besagten mikroinjizierten Keimpflanzen oder von Teilen davon ganze Pflanzen heranzieht und nach Zwischenschaltung eines oder mehrerer sexueller oder asexueller Vermehrungsschritte die so erhältliche, homogen transformierte, asexuelle oder sexuelle Nachkommenschaft besagter heterogen transformierter Ausgangspflanzen mit Hilfe bekannter Verfahren selektioniert.

2. Verfahren gemäss Anspruch 1, dadurch gekennzeichnet, dass das sich bei besagten Pflanzen um Keimlinge handelt, die sich in einem Entwicklungsstadium befinden, das sich von der beginnenden Kotyledonarentwicklung bis hin zur Sekundärblattentwicklung erstreckt.

3. Verfahren gemäss Anspruch 1, das durch die folgenden spezifischen Verfahrensschritte gekennzeichnet ist:

a) Keimung von Samen der jeweiligen Zielpflanze und Selektion geeigneter Keimungsstadien;

b) Freilegen des Apikal- und/oder Axialmeristems für die sich anschliessende Mikroinjektion;

c) Mikroinjektion einer DNA-haltigen Lösung enthaltend ein oder mehrere DNA-Fragmente in die Zellen des unter b) freigelegten Meristems;

d) Anzucht der mikroinjizierten Keimlinge zu vollständigen, heterogen transformierten Pflanzen;

e) Herstellung sexueller oder asexueller Nachkommenschaft der gemäss Punkt d) erhältlichen heterogen transformierten Pflanzen und Auslesen der homogen transformierten Nachkommen.

4. Verfahren gemäss Anspruch 1, das durch die folgenden spezifischen Verfahrensschritte gekennzeichnet ist:

a) Keimung von Samen der jeweiligen Zielpflanze und Selektion geeigneter Keimungsstadien;

b) Freilegen des Apikal- und/oder Axialmeristems für die sich anschliessende Mikroinjektion;

c) Mikroinjektion einer DNA-haltigen Lösung enthaltend ein oder mehrere DNA-Fragmente in die Zellen des unter b) freigelegten Meristems;

d₁) Isolierung und in vitro Regeneration von mikroinjizierten Apikal- und/oder Axialmeristemen ohne Selektionsdrück zu vollständigen, heterogen transformierten Pflanzen; oder

d₂) Isolierung und in vitro Regeneration von mikroinjizierten Apikal- und/oder Axialmeristemen unter Selektionsdruck zu vollständigen, homogen transformierten Pflanzen;

e) Herstellung sexueller oder asexueller Nachkommenschaft der unter Punkt d₁) erhältlichen heterogen transformierten Pflanzen und Auslese der homogen transformierten Nachkommen.

5. Verfahren gemäss einem der Ansprüche 3 oder 4, dadurch gekennzeichnet, dass der Mikroinjektionsvorgang in folgenden Teilschritten abläuft:

a) Fixieren der Keimlinge mit Hilfe einer speziellen Haltevorrichtung

b) Injektion der DNA haltigen Lösung in die Zellen des Apikal- und/oder Axialmeristems mit Hilfe der Mikroinjektionskapillare

c) Drehen der Keimlinge und erneute Injektion.

6. Verfahren gemäss Anspruch 5, dadurch gekennzeichnet, dass es sich bei den Mikroinjektionskapillaren um Mikroelektroden mit eingeschmolzenen Glasfilamenten aus Borosilikat handelt.

7. Verfahren gemäss Anspruch 5, dadurch gekennzeichnet, dass man den Mikroinjektionsvorgang 1 bis 1000 mal pro Meristem durchführt.

8. Verfahren gemäss Anspruch 5, dadurch gekennzeichnet, dass das Injektionsvolumen 1 pl bis 100 pl pro injizierter Zelle beträgt.

9. Verfahren gemäss Anspruch 5 dadurch gekennzeichnet, dass die DNA-Konzentration der injizierten DNA-haltigen Lösung zwischen 0,1 μg/μl und 10 μg/μl beträgt.

10. Verfahren gemäss Anspruch 5, dadurch gekennzeichnet, dass besagte DNA-haltige Injektionslösung die transformierende DNA in linearisierter Form enthält.

11. Verfahren gemäss Anspruch 5, dadurch gekennzeichnet, dass besagte DNA-haltige Injektionslösung die transformierende DNA in überspiralisierter Form enthält.

12. Verfahren gemäss Anspruch 5, dadurch gekennzeichnet, dass besagte DNA-haltige Injektionslösung die transformierende DNA in Form eines Gemisches aus linearisierter und überspiralisierter DNA enthält.

13. Verfahren gemäss Anspruch 5, dadurch gekennzeichnet, dass besagtes DNA-haltige Injektionslösung ein rekombinantes DNA-Molekül enthält, das im wesentlichen aus einem Strukturgen besteht, das für eine nützliche und wünschenswerte Eigenschaft kodiert und das mit in der pflanzlichen Zelle aktiven Expressionssignalen in operabler Weise verknüpft ist.

14. Verfahren gemäss Anspruch 13, dadurch gekennzeichnet, dass besagtes Strukturgen der transformierten Pflanze eine erhöhte Toleranz oder Resistenz gegenüber Pathogenen, Herbiziden, Insektiziden oder anderen Bioziden verleiht.

15. Verfahren zur Herstellung reinerbiger homozygoter Transformanten gemäss Ansprüche 3, dadurch gekennzeichnet, dass man

a) heterogen transformierte Pflanzen, die einer Selbstbestäubung zugänglich sind, d.h. die keine Selbstinkompatibilitäten aufweisen, zur Herstellung von Inzuchtlinien wiederholt mit sich selbst kreuzt; oder

c) heterogen transformierte Pflanzen, die einer Selbstbestäubung nicht unmittelbar zugänglich sind, zur Herstellung von Inzuchtlinien wiederholt mit Pflanzen kreuzt, die einen hohen Verwandschaftsgrad aufweisen.

16. Verfahren gemäss Anspruch 3, dadurch gekennzeichnet, dass die Keimlinge nach erfolgter Mikroinjektion direkt in Erde übertragen und wie normale Sämlinge zu ganzen Pflanzen herangezogen werden.

17. Verfahren gemäss Anspruch 3, dadurch gekennzeichnet, dass die Keimlinge nach erfolgter Mikroinjektion auf ein spezifisches Kultivierungsmedium überführt werden, das eine für die weitere Entwicklung des Sämlings zur ganzen Pflanze geeignete Zusammensetzung aufweist.

18. Verfahren gemäss Anspruch 4, dadurch gekennzeichnet, dass man von Pflanzen, die einer Regeneration ausgehend von isolierten Meristemen zugänglich sind, die mikroinjizierten Apikal- und/oder Axialmeristeme entweder unmittelbar nach der erfolgten Mikroinjektion oder aber zu einem beliebigen späteren Zeitpunkt mit oder ohne Selektionsdruck isoliert und diese zu morphogenenen Kulturen heranzieht, aus denen dann wiederum in vitro mit Hilfe bekannter Verfahren ganze Pflanzen regeneriert werden können.

19. Verfahren gemäss Anspruch 18, dadurch gekennzeichnet, dass die Kultivierung der isolierten mikroinjizierten Apikal- und/oder Axialmeristeme in einem Kultivierungsmedium durchgeführt wird, das die Bildung adventiver Sprossknospen oder adentiver Embryonen anregt und das mit einem osmotisch neutralen Verdickungs- oder Geliermittel versetzt ist.

20. Verfahren gemäss Anspruch 19, dadurch gekennzeichnet, dass es sich bei besagtem Verdickungsmittel um Co-Polymere aus Saccharose und Epichlorhydrin mit einem Molekulargewicht zwischen 70 000 und 400 000 handelt.

21. Verfahren gemäss Anspruch 19, dadurch gekennzeichnet, dass es sich bei besagtem Geliermittel um einen ausgewählt aus der Gruppe bestehend aus Agar, Agarose, Alginat, Pektinat, Gelrite® oder Gelatine handelt.

22. Verfahren gemäss Anspruch 19, dadurch gekennzeichnet, dass das Kulturmedium Phytohormone aus der Gruppe der Auxine und der Cytokinine in einer Konzentration von jeweils 0,1 mg/l bis 20.0 mg/l enthält, abhängig vom jeweiligen Entwicklungsstand der morphogenen Kultur.

23. Verfahren gemäss Anspruch 19, dadurch gekennzeichnet, dass der pH-Wert des Kulturmediums zwischen pH 5.2 und pH 6.5 beträgt.

24. Verfahren gemäss Anspruch 19, dadurch gekennzeichnet, dass die Kultivierungsdichte der Meristeme zwischen 1 Meristem $\mu$l Kulturmedium und 50 Meristemen $\mu$l Kulturmedium beträgt.

25. Verfahren gemäss Anspruch 19, dadurch gekennzeichnet, dass die Kultivierung der Meristeme zumindest zeitweise in einem Zweikompartimenten-System durchgeführt wird.

26. Verfahren gemäss Anspruch 4, dadurch gekennzeichnet, dass man die Meristemkulturen, wenn sie ein bestimmtes Entwicklungsstadium erreicht haben zur Differenzierung der Spross- und Wurzelscheitel auf feste Kulturmedien, die üblicherweise für die Regeneration von Pflanzen verwendet werden, überführt.

27. Verfahren gemäss Anspruch 4, dadurch gekennzeichnet, dass die Kulturmedien eine Selektivsubstanz enthalten, die eine positive Selektion der transgenen Zellen ermöglicht.

28. Verfahren gemäss Anspruch 4, dadurch gekennzeichnet, dass man die regenerierten Pflänzchen in Erde pflanzt und in gleicher Weise wie normale Sämlinge weiterbehandelt.

29. Verfahren zur Herstellung transgener Pflanzen gemäss Anspruch 1.

30. Verfahren zur Herstellung transgener Pflanzen sowie deren sexueller und asexueller Nachkommenschaft gemäss Anspruch 1, dadurch gekennzeichnet, dass man diejenigen Pflanzen selektioniert, bei denen ein Grossteil der somatischen Zellen und oder der Keimzellen transformiert sind.

31. Verfahren zur Herstellung von transgenem Saatgut von Pflanzen, die gemäss einem Verfahren nach Anspruch 1 transformiert worden sind, dadurch gekennzeichnet, dass man besagte Pflanzen sexuell vermehrt und die sich entwickelnden Samen, die noch das eingeschleuste genetische Material enthalten, mit Hilfe bekannter Verfahren gewinnt.

32. Verfahren zur Herstellung transformierter, lebensfähiger Teile von Pflanzen, die gemäss einem Verfahren nach Anspruch 1 transformiert worden sind, dadurch gekennzeichnet, dass man ausgehend von besagten Pflanzen mit Hilfe bekannter Methoden transformierte Pflanzenteile ausgewählt aus der Gruppe bestehend aus Protoplasten, Zellen, Zellklonen, Zellagglomeraten, Kallus- und/oder Gewebekulturen, Samen, Pollen, Eizellen, Zygoten oder Embryonen gewinnt und diejenigen selektioniert, die noch das eingeschleuste genetische Material enthalten.

33. Verfahren zur Herstellung von Kreuzungs- und Fusionsprodukten mit dem in den Ansprüchen 30 bis 32 definierten transformierten Pflanzenmaterial, die noch das eingeschleuste genetische Material enthalten, dadurch gekennzeichnet dass man besagtes Pflanzenmaterial untereinander oder mit Fremdmaterial kreuzt oder fusioniert und diejenigen Kreuzungs- und Fusionsprodukte selektioniert, die noch das eingeschleuste genetische Material enthalten.

34. Verfahren zur Herstellung von Varianten und Mutanten von transgenen Pflanzen, die gemäss einem Verfahren nach Anspruch 1 transformiert worden sind, dadurch gekennzeichnet, dass man besagte Pflanzen oder lebensfähige Teile davon sowie deren sexuelle und asexuelle Nachkommenschaft mit Hilfe bekannter Methoden mutiert und anschliessend dasjenige Material selektioniert, das noch das eingeschleuste genetische Material enthält.

35. Herstellung transgener Pflanzenteile aus Pflanzen, die gemäss einem Verfahren nach Anspruch 1 transformiert worden sind, wie z.B. Blüten, Stengel, Früchte, Blätter, Wurzeln, dadurch gekennzeichnet, dass man besagte Pflanzenteile isoliert und diejenigen selektioniert, die noch in einem Grossteil ihrer Zellen das eingeschleuste genetische Material enthalten.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 738 874 (INSTITUT BOTANIKI IMENI N.G. CHOLODNOGO AKADEMIE, KIEV) * Beispiel 7 * | 30-35 | C 12 N 15/82 A 01 H 5/00 C 12 N 5/10 |
| Y | | 1-29 | |
| | --- | | |
| Y | JOURNAL OF CELLULAR BIOCHEMISTRY, Suppl. 13D, 27. März - 7. April 1989, Seite 266, Zusammenfassung M143, Alan R. Liss, New York, US; J. OARD et al.: "High velocity microprojectile delivery of foreign genes and their expression in apices of Triticum aestivum" * Zusammenfassung M143 * | 1-29 | |
| | --- | | |
| X | EP-A-0 301 749 (AGRACETUS) * Ganze Dokument besonders Beispiel 8 * | 30-35 | |
| Y | | 3,15-17 | |
| | --- | | |
| X | IN VITRO CELLULAR & DEVELOPMENTAL BIOLOGY, Band 24, Nr. 9, September 1988, Seiten 951-954, Tissue Culture Association Inc.; E.C. ULIAN et al.: "Transformation of plants via the shoot apex" * Ganze Dokument * | 30-35 | **RECHERCHIERTE SACHGEBIETE (Int. Cl.5)** C 12 N A 01 H |
| Y | IDEM | 4,18,27 ,28,26 | |
| | --- -/- | | |

Der vorliegende Recherchenbericht wurde für alle Patentansprüche erstellt

| Recherchenort | Abschlußdatum der Recherche | Prüfer |
|---|---|---|
| DEN HAAG | 28-08-1990 | MADDOX A.D. |

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | THEOR. APPL. GENETICS, Band 75, Dezember 1987, Seiten 30-36, Springer-Verlag; G. NEUHAUS et al.: "Transgenic rapeseed plants obtained by the microinjection of DNA into microspore-derived embryoids" * Seite 31, linke Spalte * | 30-35 | |
| Y | IDEM | 5-14 | |
| A | IDEM | 19-25 | |
| X | BIOTECHNOLOGY, Band 6, August 1988, Seiten 923-926; D.E. McCABE et al.: "Stable transformation of soybean (Glycine max) by particle acceleration" * Seite 923, rechte Spalte * | 30-35 | |
| Y | IDEM | 19,21-23 | |
| X | EP-A-0 290 395 (SANDOZ) * Anspruch 6 * | 30-35 | RECHERCHIERTE SACHGEBIETE (Int. Cl.5) |
| A | BIOTECHNOLOGY, Band 6, April 1988, Seiten 397-402; I.K. VASIL: "Progress in the regeneration and genetic manipulation of cereal crops" * Seite 400, rechte Spalte, letzter Absatz - Seite 401, linke Spalte, Absatz 1 * | 1-5,15, 30-35 | |
| A | CELL CULTURE AND SOMATIC CELL GENETICS OF PLANTS, Band 1, 1984, Seiten 43-48, Academic Press, Inc.; H. BINDING: "Clonal propagation: shoot cultures" * Ganze Dokument * | 19,21-23 | |

-/-

Der vorliegende Recherchenbericht wurde für alle Patentansprüche erstellt

| Recherchenort | Abschlußdatum der Recherche | Prüfer |
|---|---|---|
| DEN HAAG | 28-08-1990 | MADDOX A.D. |

EPO FORM 1503 03.82 (P0403)

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

................................................................

& : Mitglied der gleichen Patentfamilie, übereinstimmendes Dokument

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 267 159  (CIBA-GEIGY) <br> * Ansprüche 11-13,17,62-69 * <br> --- | 1,30-35 ,2 | |
| O,A | JOURNAL OF CELLULAR BIOCHEMISTRY, Suppl. 13D, 27. März - 7. April 1989, Seite 228, Zusammenfassung Nr. M 001, Alan R. Liss, Inc., New York, US; I. POTRYKUS et al.: "Approach to cereal transformation via microinjection into microspore-derived and into zygotic proembryos" <br> * Ganze Zusammenfassung * <br> --- | 1,30-35 | |
| P,X | EP-A-0 331 083  (CIBA-GEIGY) <br> * Ansprüche 1,14,51-58 * <br> ----- | 30-35,1 ,29 | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| DEN HAAG | 28-08-1990 | MADDOX A.D. |

EPO FORM 1503 03.82 (P0403)